# EUROPEAN PATENT APPLICATION

(11) **EP 2 446 898 A1**
(43) Date of publication of application: **02.05.2012**
(21) Application number: 10382259.9
(22) Date of filing: 30.09.2010
(51) Int. Cl.: A61K 38/27, A61P 31/18

(54) **Use of growth hormone to enhance the immune response in immunosuppressed patients**

(71) Applicant: Laboratorios del. Dr. Esteve, S.A., 08041 Barcelona (ES); Fundació Privada Institut de Recerca de la SIDA-Caixa, 08916 Badalona (ES); Institució Catalana De Recerca I Estudis Avançats (ICREA), 08010 Barcelona (ES)
(72) Inventor: Bofill Soliguer, Margarida, 08912 Badalona (ES); García Alcaide, Felipe, 08041 Barcelona (ES)
(74) Representative: ABG Patentes, S.L.

(57) **Abstract**

The present invention relates to a method for stimulating the recovery of cellular and humoral responses *in vivo* based on growth hormone.

## Description

### FIELD OF THE INVENTION

The invention relates to the field of immunology and, more in particular, to methods for improving the immune response against antigens in immunosuppressed patients.

### BACKGROUND OF THE INVENTION

It is estimated that more than 60 million people worldwide have been infected by the human immunodeficiency virus since 1982. Nearly half of these infected individuals have died of the resultant Acquired Immunodeficiency Syndrome (AIDS) during the same time frame. Although the virus spread seems to have reached a plateau lately, 2.7 million HIV new infections were reported in 2007. HIV still is a major public health problem. *See* UNAIDS, 2008 Report on the Global AIDS Epidemic.

One of the main problems afflicting immunocompromised individuals is the loss specific humoral responses acquired prior to the event triggering the immunosuppression (e.g. HIV infection, chemotherapy). In other words, these patients lose their capacity to act against antigens for which they already had an immune response. Also important in the treatment of these types of patients is the boosting of their cellular immune response. This could have the effect of improving their capability to respond against a broad spectrum of potentially damaging infections.

It has been reported that adults who had been severely immunosuppressed during chemotherapy (e.g. bone marrow transplantation, cancer treatment) or through HIV infection, have a restricted and clonal T-cell repertoire. *See* Douek D, et al., Nature 1998; 396(6712):690-695. There is frequently a loss of specific immune responses to HIV as well as other antigens at the beginning of the HIV infection. *See* Shearer G, et al., AIDS 1991; 5(3):245-253. This situation has been noticed even in patients subject to highly active antiretroviral therapy (HAART). *See* Lederman H, et al., J. Infect. Dis. 2003; 188(12):1794-1803; Teixeira L, et al., AIDS 2001; 15(14):1749-1756. It is possible that the thymus may be involved in the maintenance of the T-cell repertoire. Consequently, the regulation of this gland could yield unforeseen immunotherapeutic benefits.

Several strategies have been proposed to reconstitute and improve thymus function. *See* Berzins S, et al., Trends Mol. Med. 2002; 8(10):469-476. For instance, the administration of recombinant pituitary growth human hormone to children and adults with GH deficiencies and to HIV infected subjects has been described. *See* McCune J, et al., J. Clin. Invest. 1998; 101(11):2301-2308; Imami N, et al., AIDS Res. Hum. Retrovir. 1999; 15(17):1499-1508; Napolitano L, et al., AIDS 2002; 16(8):1103-1111; Morrhaye G, et al., PLoS One 2009; 4(5):e5668; Al-harti L, et al., J. Hermatother. Stem Cell Res. 2002; 11(5):777-786 and Savino W, et al., Ann. NY Acad. Sci. 2000; 917:748-754. In addition, the administration of recombinant growth hormone to HIV subjects with lypodistrofia, or patients with persistent low CD4 T-cell counts (ACTG), has resulted in an increase in thymic volume and activity levels. *See* Herasimtschuck A, et al., J. Immune Based Ther. Vacc. 2008; 6:7 and Napolitano L, et al., J. Clin. Invest. 2008; 118(3):1085-1098; Pires A, et al., Antivir. Ther. 2004; 9(1):67-75. An expansion of CD4 T-cells, and to a lesser extent CD8 T-cells, has been elicited in these cases. *See* Napolitano *et al.*, 2002 and 2008, Teixeira *et al.*, 2001 and Imami *et al.,* 1999, *supra.* Finally, growth hormone therapy has also been shown to stimulate the generation of bone marrow hematopoietic cells, including T- and B-cell precursors. *See* French R, et al., Endocrinology 2002; 143(2):690-699. However, the effective use of growth hormone to stimulate the recovery of specific humoral responses *in vivo* has not been described yet in the art.

### SUMMARY OF THE INVENTION

In a first aspect, the invention relates to an agent capable of increasing the activity of growth hormone for use in a method of increasing the immune response against an antigen in a subject.

In a second aspect, the invention relates to a composition or kit comprising:
(i) An agent capable of increasing the activity of growth hormone and
(ii) An antigen in amounts sufficient to induce an immune response.

In further aspects, the invention relates to a composition or kit according to the invention for use in medicine as well as to a composition or kit according to any of claims for use as a vaccine.

### BRIEF DESCRIPTION OF THE FIGURES

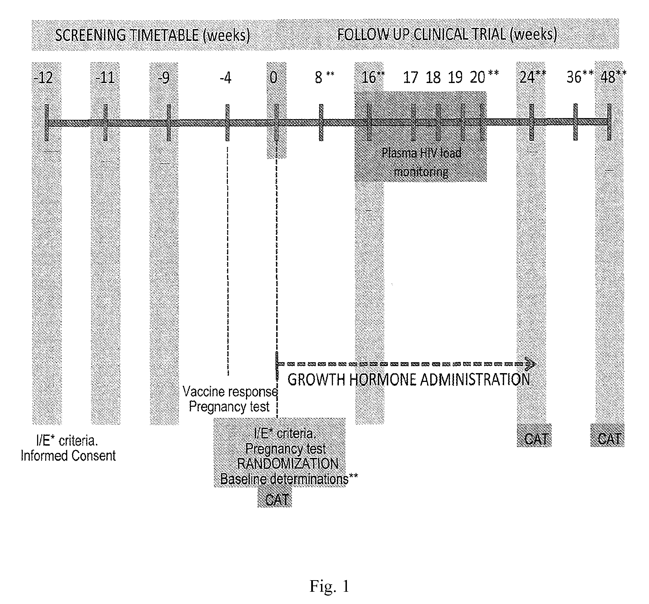

**Figure 1****.** Trial diagram. Twenty-one HIV positive subjects were randomized in three groups A, B and C). Group A and group B received recombinant growth hormone for 6 months. In addition, the group A was vaccinated against hepatitis A, hepatitis B and tetanus toxoid 2 months after the initiation of treatment. Group C was vaccinated against these antigens but not treated with growth hormone. Seven HIV negative subjects were used as controls for the vaccines. All HIV positive subjects were on HAART.

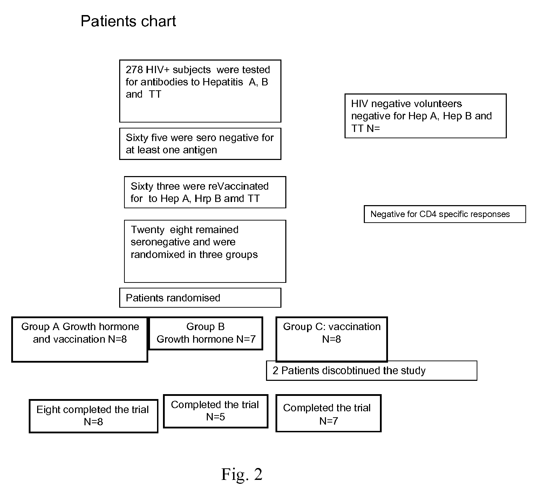

**Figure 2****.** Chart of patients enrolled in the trial.

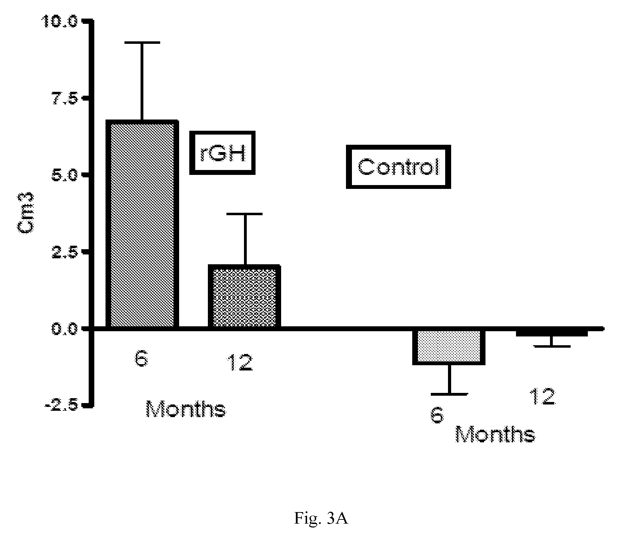

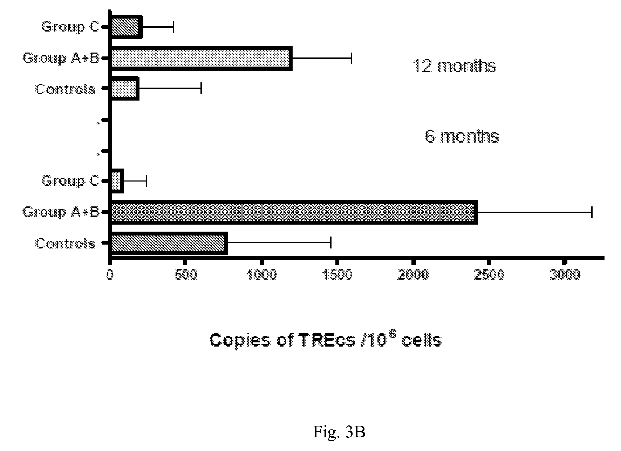

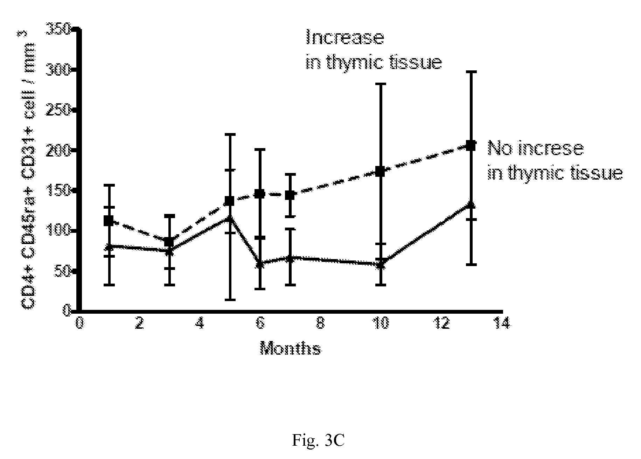

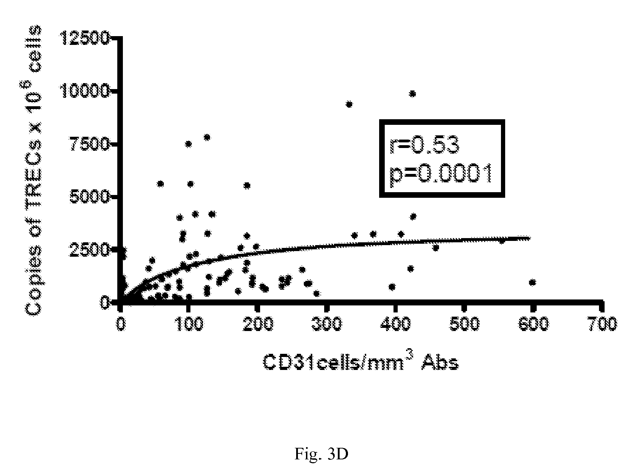

**Figure 3****.** Impact of growth hormone on the reconstitution of the thymus. Twenty-one HIV positive individuals were treated with recombinant growth hormone (rGH) for 6 months. A chest computerized tomograms (CAT) was performed to measure changes in volume and density of the thymic tissue from 20 subjects. **A:** The subjects were tested at months 0 (baseline), 6 and 12. The bars show the changes in volume (cm³) from 15 HIV infected patients that received rGH and 7 patients that did not at the end of the treatment and 6 and 12 months afterwards. The thymic tissue receded when the treatment was stopped, but was still larger than its original size 6 months after the trial. **B:** The graphs show an increase in the levels of TRECs in patients that received the recombinant growth hormone (Group A + B) compared to those that had only received vaccination. (Group C) and the HIV negative group (N=7) at 6 and 12 months after the initiation of the trial. The results are expressed in copies of TRECs /10⁶ cells. **C:** The graph shows the absolute numbers of CD4, CD45 and CD31 recent thymic emigrants in patients that had at least a two-fold increase in thymic tissue after treatment with rGH (broken line) and those that did not (continuous line), measured at baseline and at two-month periods. There was a statistically significant difference between the two groups (p<0.01). **D:** The diagram shows a statistically significant correlation between the levels of TCR excision circles (TRECs) and the absolute numbers of CD4, CD45RA and CD31 (cells/mm³) from the subjects enrolled in the trial.

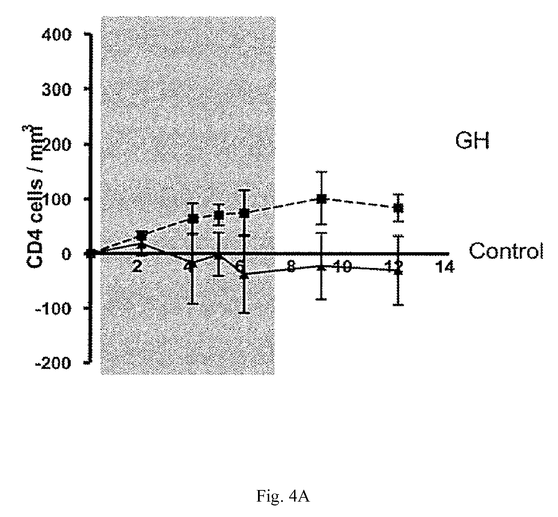

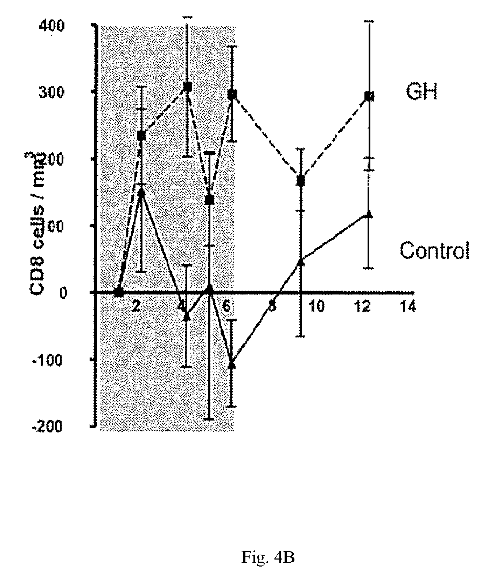

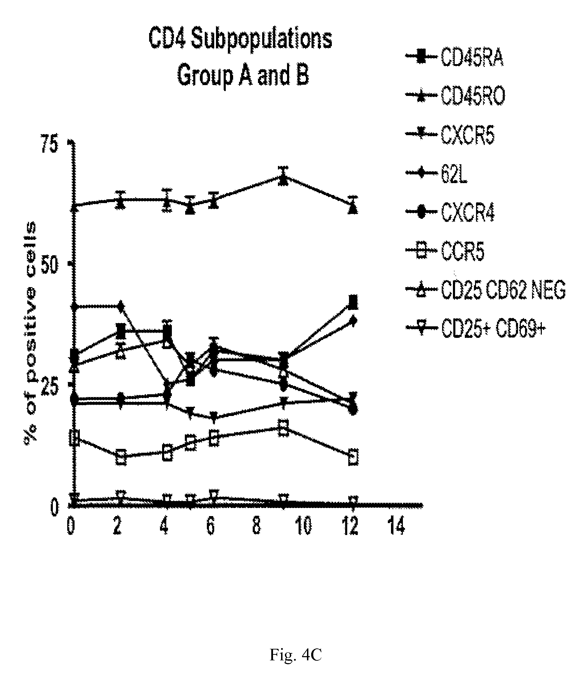

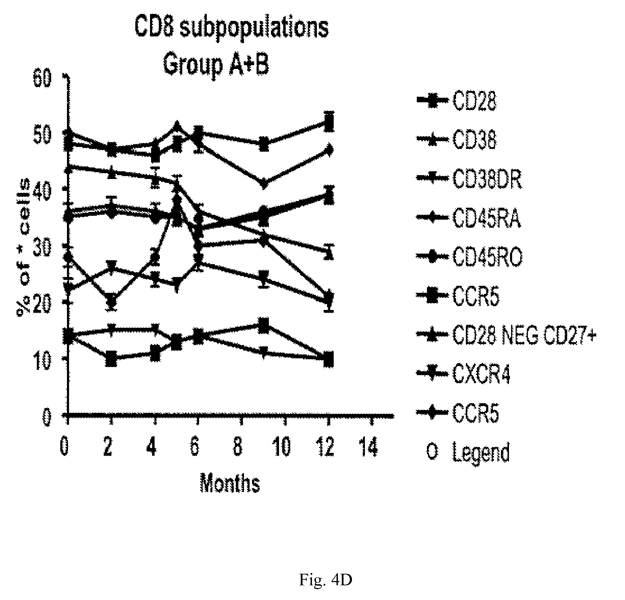

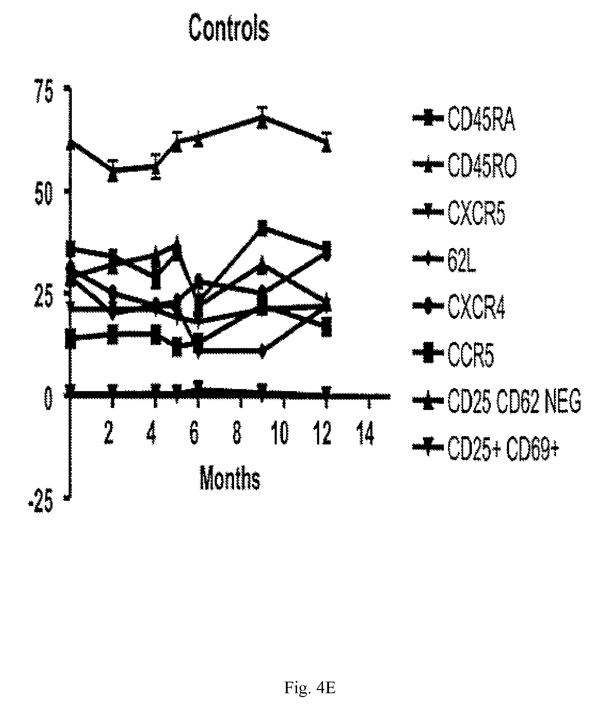

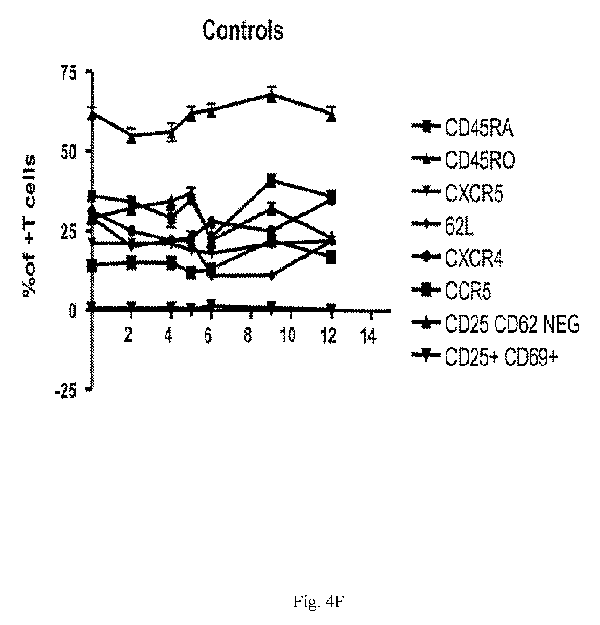

**Figure 4****.** Effect of growth hormone treatment on CD4 and CD8 subsets. Thirteen HIV positive individuals were treated with recombinant growth hormone for 6 months (grey). The absolute numbers of CD4 (**A**) and CD8 (**B**), and the percentage levels of the CD4 and CD8 subsets, were measured at months 0 (base line), 2, 4, 5, 6 ,9 and 12. Panels A and B show the mean + standard error of the values of the absolute CD4 and CD8 T-cells from patients that had received recombinant growth hormone (continuous line) and those who did not (broken line). Panels C and D show the percentages of CD4 and CD8 subpopulation of patients receiving GH (C and D) and those that did not (E and F).

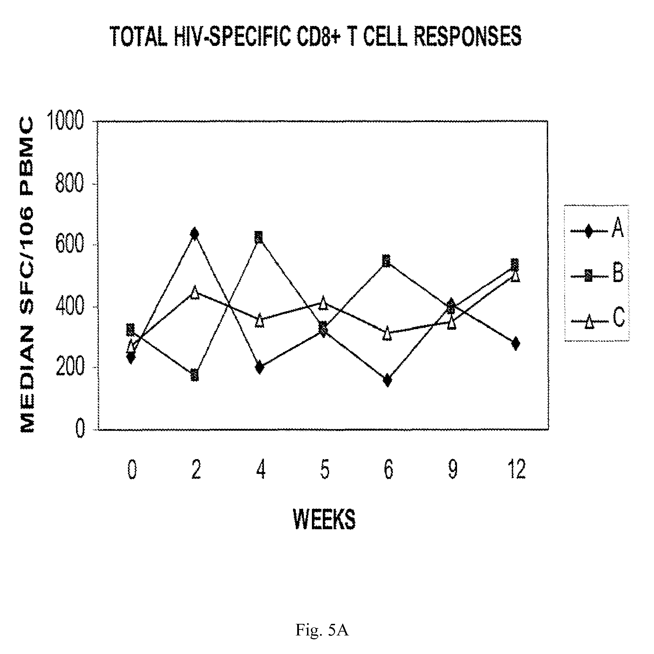

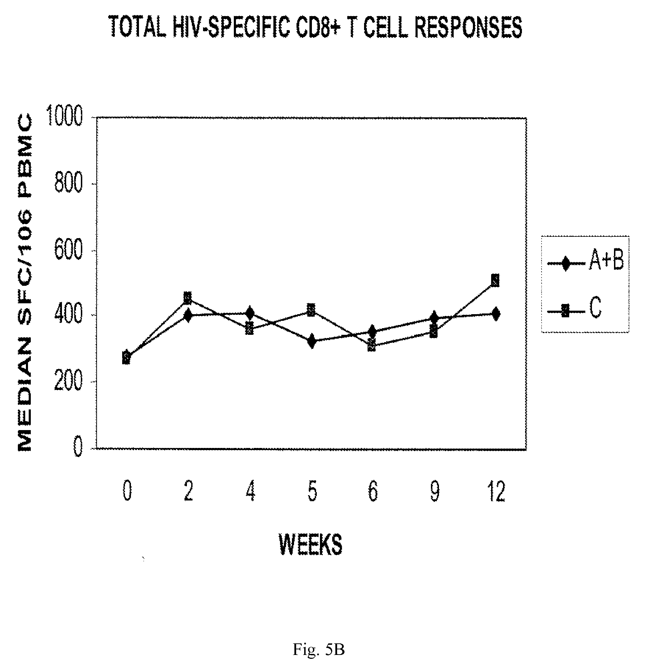

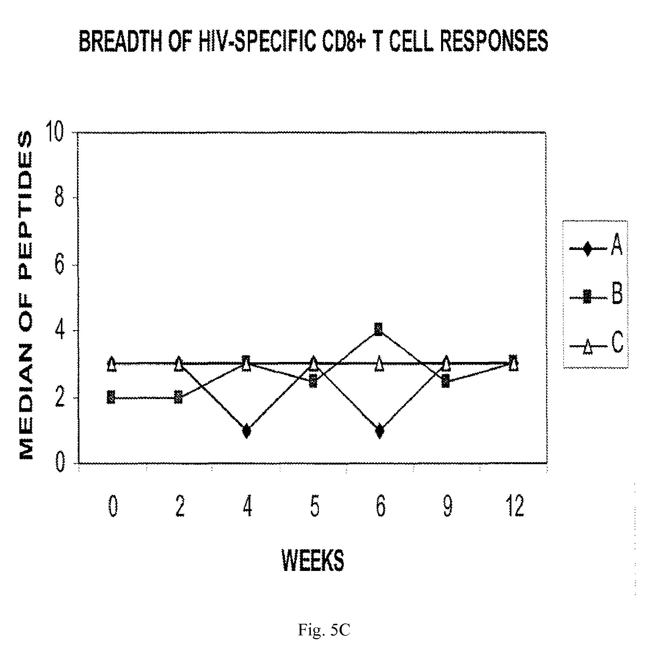

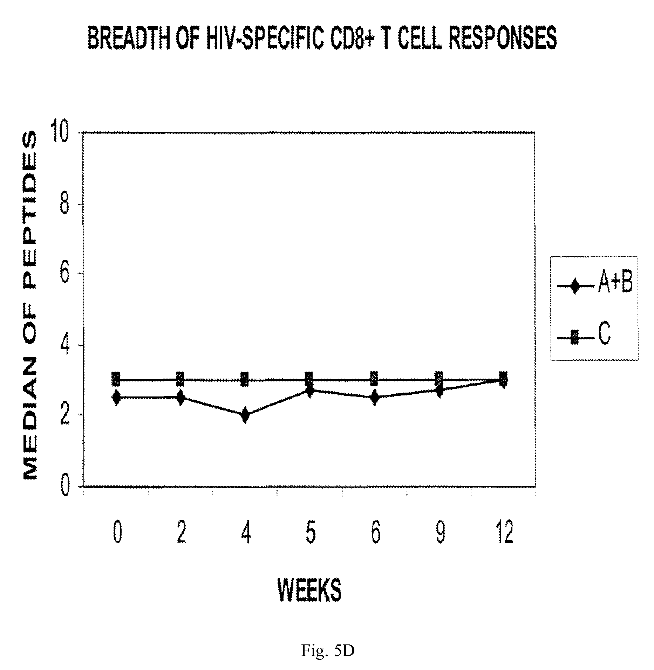

**Figure 5****.** Effect of growth hormone treatment of CTL responses to HIV. **A, B:** Median of total IFN-γ-producing CD8 T-cells against HIV peptides. The median of the sum of all considered positive responses in the different arms of the study (A, B or A+B, and C) are represented in the graph as median SFC/106 PBMC at the different time-points analyzed during the follow-up. **C, D:** Median breadth of the HIV-specific CD8 T-cell responses. The median of the positive peptides in the different arms of the study (A, B or A+B, and C) are represented in the graph as median of peptides at the different time-points analyzed during the follow-up.

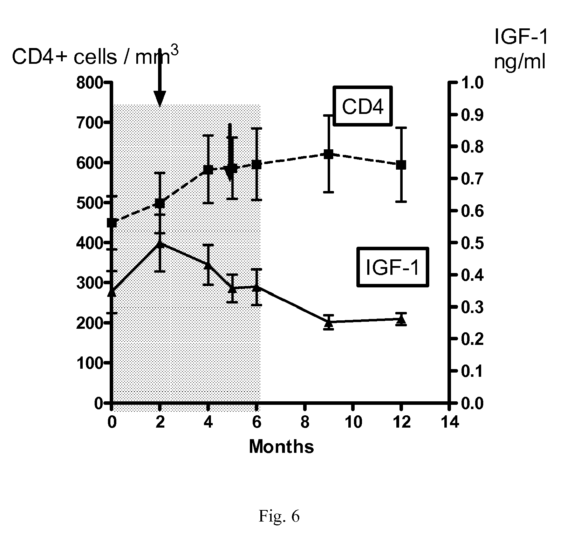

**Figure 6****.** Correlation between the levels of insulin like growth factor 1 and CD4 counts. Twenty-one HIV positive individuals were treated with rGH for 6 months (grey). The absolute numbers of CD4 and levels of insulin like growth factor 1 (IGF-1) were measured at months 0 (base line), 2, 4, 5, 6 9 and 12. The figure shows the mean numbers of absolute CD4 T-cells from patients that had received rGH and the IGF-1 levels. There is no direct correlation between the IFG-1 levels and the increase in CD4 levels.

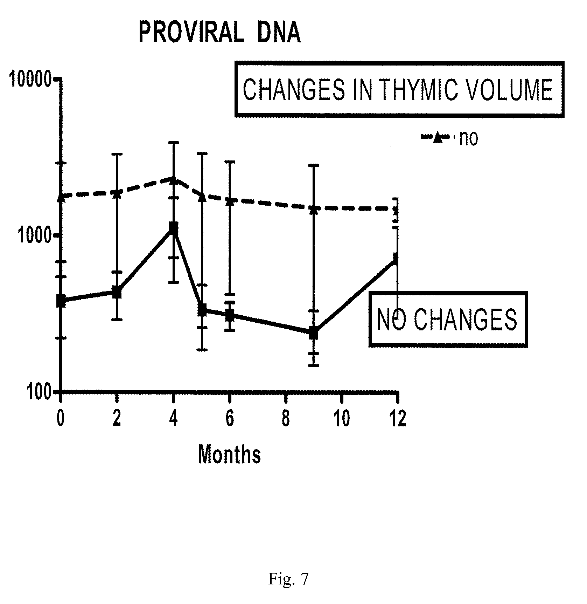

**Figure 7****:** shows the variations in thymic volume during and after treatment with rGH.

### DETAILED DESCRIPTION OF THE INVENTION

### Therapeutic methods of the invention

The present invention proposes the use of growth hormone to stimulate the recovery of cellular and humoral responses *in vivo.* The hormone has been demonstrated to be effective in inducing the recovery of specific humoral against various pathogens such as hepatitis A, hepatitis B and tetanus toxoid in HIV patients. In addition, growth hormone is useful to enhance cellular immune response by the activation, or selective expansion, of T-cell subpopulations such as the CD4 follicular helper T-cells, which are essential for T-cell dependent responses.

Thus, in a first aspect, the invention relates to an agent capable of increasing the activity of growth hormone for use in a method of increasing the immune response against an immunogenic protein in a subject. Alternatively, the invention relates to the use of an agent capable of increasing the activity of growth hormone for the preparation of a medicament for increasing the immune response against an immunogenic protein in a subject. Alternatively, the invention relates to a method of increasing the immune response against an immunogenic protein in a subject comprising administering to said subject an agent capable of increasing the activity of growth hormone.

### Agent capable of increasing the activity of growth hormone

The term "agent", as used herein, includes a compound that induces a desired pharmacological and/or physiological effect. The term also encompass pharmaceutically acceptable and pharmacologically active ingredients of those compounds including but not limited to their salts, esters, amides, prodrugs, active metabolites and analogs. When the above term is used, then it is to be understood that this includes the active agent *per se* as well as its pharmaceutically acceptable, pharmacologically active salts, esters, amides, prodrugs, metabolites and analogs. The term "agent" is not to be construed narrowly but extends to small molecules, proteinaceous molecules such as peptides, polypeptides and proteins as well as compositions comprising them and genetic molecules such as RNA, DNA and mimetics and chemical analogs thereof as well as cellular agents. The term "agent" includes a cell which is capable of producing and secreting the polypeptides referred to herein as well as a polynucleotide comprising a nucleotide sequence that encodes this polypeptide. Thus, the term "agent" extends to nucleic acid constructs including vectors such as viral or non-viral vectors, expression vectors and plasmids for expression in and secretion in a range of cells. The term "agent" includes plasma or other blood products.

The term "capable of increasing the activity of growth hormone", as used herein, refers to any compound which is capable of inducing an increase in the activity of GH in a cell or organism. The enhanced activity could be caused, for instance, by an increase in transcription of the GH gene, by an increase in the translation of the GH mRNA or by a decrease in the degradation of the GH mRNA.

In a preferred embodiment, the agent capable of increasing the activity of GH is not a GH secretagogue. The term "GH secretagogue", as used herein, refers to any compound or molecule, natural or synthetic, which may result in, either directly or indirectly, GH secretion and/or an increase in GH secretion and include, without limitation, GRF and variants thereof, GHRH and variants thereof, ghrelin and variants thereof, L-163,540, L-692-429, L-692-585 and MK-677 (Merck & Co., Whitehouse Station, NJ, USA); EP01572 (Ardana Biosciences, Edinburgh, UK); GHRP-1, GHRP-2 and GHRP-6 (Bower C, *et al.*,); Hexarelin (Europeptide, Brussels, BE); G-7039 and G-7502 (Genentech, San Francisco, CA, USA); Ipamorelin, NN703, NNC 26-0235, NNC26-0722 and NNC26-0610 (Novo Nordisk, Bagsværd, DK); CP-424,391 (Pfizer, New York, NY, USA); EP51319 (Theratechnology, Montréal, QB, CA); RC-1291 (Rejuvenon, Houston, TX, USA) and BIM28125 and BIM28131 (Ipsen, Paris, FR).

In a preferred embodiment, the agent capable of increasing the activity of GH is selected from the group consisting of:
(i) growth hormone or a functionally equivalent variant thereof,
(ii) a polynucleotide encoding growth hormone or a functionally equivalent variant thereof and
(iii) a cell secreting growth hormone or a functionally equivalent variant thereof.

### Growth hormone or a functionally equivalent variant thereof

The term "growth hormone (GH)" refers generally to growth hormones secreted by the pituitary gland in mammals. Although not an exhaustive list, examples of mammals include human, apes, monkey, rat, pig, dog, rabbit, cat, cow, horse, mouse, rat and goat. According to a preferred embodiment of this disclosure, the mammal is a human.

"Human growth hormone (hGH)" denotes a protein having an amino acid sequence, structure and function characteristic of native human growth hormone. As used herein, human growth hormone (hGH) also includes any isoform of native human growth hormone, including but not limited to, isoforms with molecular masses of 5, 17, 20 (hereinafter hGH-20K or 20K), 22 (hereinafter hGH-22K or 22K), 24, 36 and 45 kDa. *See* Haro L, et al., J. Chromatography B 1998; 720:39-47. Thus, the term hGH includes the 191 amino acid sequence of native hGH, somatotropin, and the 192 amino acid sequence containing an N-terminal methionine (Met-hGH) and somatrem. *See* Goeddel D, et al., US 4,342,832; Dalboge H, et al., US 5,633,352.

The term "functionally equivalent variant", when referring to GH, as used herein, refers to any variant polypeptide obtained by deletion, insertion or mutation of one or more amino acids from the native GH sequence and which maintains substantially one or more of the biological activities of GH. Typically, functionally equivalent variants of GH include but are not limited to GH analogs, GH isoforms, GH mimetics, GH fragments, hybrid GH proteins, fusion proteins, oligomers and multimers, homologues, glycosylation pattern variants, variants, splice variants and muteins thereof.

Suitable means for determining whether a given polypeptide is a functionally equivalent variant GH can be any of the methods commonly known to the skilled person including, without limitation, the method described in example 1 of the present invention based on the ability of the suspected variant to promote an increase of thymic volume (measured as TAC), levels of recent thymic emigrants (measured as the number T-cell receptor excision circle or TRECS) and absolute CD4 and CD8 T-cell counts (measured *inter alia*, by FACS), the method shown in example 2 of the present invention based on the determination of the ability of the suspected variant to induces the recovery of the CD4 specific responses to hepatitis A/B, TT and HIV p24 in subjects that were undetectable at baseline, a radioreceptor binding assay by measuring the binding between a radio-labeled hGH receptor and a mutant human growth hormone of the present invention. *See* Tsushima T, et al., J. Clin. Endocrinol. Metab. 1973; 37:334-337, Aviv H, et al., US 4,871,835; Randawa Z, et al., US 5,079,230. The ability of a variant growth hormone to promote cell growth is assessed by methods such as the tibia test as and methods based on the ability of the GH variant to cause hGH-dependent tyrosine phosphorylation in IM-9 cells. *See* Parlow A, et al., Endocrinol. 1965; 77:1126-1134; Aviv H, *et al.*, *supra.* The level of tyrosine phosphorylation of cellular proteins upon exposure to the mutant human growth hormone is shown by a monoclonal antibody against phosphorylated tyrosine. *See* Silva C, et al., Endocrinol. 1993; 132:101-108; Chihara K, US 6,238,915.

Suitable functionally equivalent variants of GH for use in the methods of the present invention include, without limitation:
1) The "basic hGH" variant (hGH-V) expressed by the placenta during pregnancy which is a product of a separate gene; like the 22 kDa hGH polypeptide it consists of 191 amino acid residues, but 13 amino acid residues at various positions in the sequence differ from those in 22 kDa hGH. *See* Bewley T, et al., Adv. Enzymol. 1975; 42:73-166; Frankenne F, et al., J. Clin. Endocrin. Metabol. 1988; 66:1171-1180; Seeburg P, DNA 1982; 1:239-249; Seeburg P, US 4,446,235; Seeburg P, US 4,670,393.
2) The 20-kDa hGH containing a deletion of residues 32-46 of hGH. *See* Kostyo J, et al., Biochem. Biophys. Acta 1987; 925:314-324; Lewis U, et al., J. Biol. Chem. 1978; 253:2679-2687.
3) Any of the numerous hGH variants, arising from post-transcriptional, post-translational, secretory, metabolic processing and other physiological processes. *See* Baumann G, Endocrine Rev. 1991; 12:424-449.
4) Derivatives which differs by at least about 1% but not by more than about 20% from the amino acid sequence of the 191 amino acid sequence of hGH or the 192 amino acid-sequence of Met-hGH while maintaining a biological activity and/or a chemical and/or physical property of the 191 amino acid hGH or the 192 amino acid Met-hGH amino acid sequence. For example, the derivative can differ by about 1% to about 20%, about 2% to about 15%, or about 5% to about 10% from the 191 amino acid sequence of hGH or the 192 amino acid-sequence of Met-hGH; the protein can differ by about 1%, about 2%, about 3%, about 4%, about 51%, about 6%, about 7%, about 8%, about 9%, about 10%, about 11%, about 12%, about 13%, about 14%, or about 15% from the 191 amino acid sequence of hGH or the 192 amino acid-sequence of Met-hGH. The differences between the derivative and the 191 amino acid hGH or the 192 amino acid Met-hGH amino acid sequence can be one or more substitutions (e.g. conservative or non conservative substitutions), deletions, additions (e.g. insertions or amino- or carboxy-terminal additions), modifications, or combinations thereof. In some embodiments, the derivative.
5) hGH analogues wherein all four of the amino acid residues at positions 172, 174, 176 and 178, respectively, are replaced by a set of four amino acid residues (in the given respective order) chosen among the following: (R, S, F, R); (R, A,Y, R); (K, T, Y, K); (R, S, Y, R); (K, A, Y, R); (R, F, F, R); (K, Q, Y, R); (R, T, Y, H); (Q, R, Y, R); (K, K, Y, K); (R, S, F, S) and (K, S, N, R). *See* Garrard L, et al., WO1992/009690.
6) hGH muteins with a set of substitutions chosen among the following: R167N, D171S, E174S, F176Y and I179T; R176E, D171S, E174S and F176Y; F10A, M14W, H18D and H21N; F10A, M14W, H18D, H21N, R167N, D171S, E174S, F176Y and I179T; F10A, M14W, H18D, H21N, R167N, D171A, E174S, F176Y and I179T; F10H, M14G, H18N and H21N; F10A, M14W, H18D, H21N, R167N, D171A, T175T and I179T; and F10I, M14Q, H18E, R167N, D171S and I179T. *See* Cunningham B, et al., US 6,143,523.
7) hGH mutein with a set of substitutions chosen among the following H18A, Q22A, F25A, D26A, Q29A, E65A, K168A and E174A and wherein G120 is further substituted with another amino acid residue, like for instance R, K, W, Y, F or E. *See* Cunningham B, et al., US 6,004,931.
8) hGH mutein with a set of substitutions chosen among H18D, H21N, R167N, K168A, D171S, K172R, E174S and I179T. *See* Cunningham B, et al., US 5,849,535.
9) hGH mutein with a set of substitutions chosen among H18D, H21N, R167N, K168A, D171S, K172R, E174S and I179T, and wherein G120 is further substituted with another amino acid residue such as R, K, W, Y, F or E. *See* Cunningham B, et al., US 6,057,292.
10) hGH mutein with a set of substitutions chosen among H18D, H21D, R167N, K168A, D171S, K172R, E174S and I179T; or H18A, Q22A, F25A, D26A, Q29A, E65A, K168A and E174A. *See* Cunningham B, et al., WO1997/011178.
11) hGH muteins with a set of substitutions chosen among K168A and E174A; R178N and I179M; and K172A and F176A. *See* Wells J, Cunningham B, WO1990/004788.
12) Deamidated or sulfoxidated forms of hGH, N-alpha-acetylated hGH-22K, Asn152-desamido-hGH-22K or Gln-137-desamido-hGH-22K.
13) Dimers and higher oligomers of hGH.
14) hGH conjugates to a molecule, such as an albumin (e.g. human serum albumin), or a water-soluble polymer, such as a polyethyleneglycol (PEG). *See* Balance D, WO1997/024445; Finn R, et al., WO2003/044056.
15) Non-covalent homo- and heterooligomers of hGH such as disulfide oligomers of hGH, covalently linked hGH, 22K-GHBP complex, 22K-alpha2-macroglobulin complex, 20K-GHBP complex, 20K-alpha2-macroglobulin complex, hGH-V_GHBP complex and hGH-22K.
16) Recombinant growth hormones for use in the present invention are any of the growth hormones selected from the list consisting of Genotropin™ (Pfizer, New York, NY, USA), Nutropin™ and Protropin™ (Genentech, San Francisco, CA, USA), Humatrope™ (Eli Lilly, Indianapolis, IN, USA), Serostim™ (Merck Serono, Geneva, CH) and Norditropin™ (Novo Nordisk, Bagsvzerd, DK). Additionally, an analogue with an additional methionine residue at the N-terminal end is also marketed as, for example, Somatonorm™ (Pfizer, New York, NY, USA).

Functionally equivalent variants of the GH suitable for use in the present invention include polypeptides showing a substantial sequence identity with any of the native GH mentioned above. Preferably, the variant GH include, polypeptides showing at least 25%, at least 40%, at least 60%, at least 70%, at least 80%, at least 90%, at least 95%, at least 96%, at least 97%, at least 98% or at least 99% identity with respect to the native GH.

"Percentage of sequence identity" is determined by comparing two optimally aligned sequences over a comparison window, wherein the portion of the polynucleotide sequence in the comparison window may comprise additions or deletions (i.e. gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The terms "identical" or "percent identity", in the context of two or more nucleic acids or polypeptide sequences, refer to two or more sequences or sub-sequences that are the same or have a specified percentage of amino acid resides or nucleotides that are the same, when compared and aligned for maximum correspondence over a comparison window, or designated region as measured using one of the following sequence comparison algorithms or by manual alignment and visual inspection. Such sequences are then said to be "substantially identical." This definition also refers to the complement of a test sequence. Optionally, the identity exists over a region that is at least about 50 amino acids or nucleotides in length, or more preferably over a region that is 75-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, for instance, by the Smith-Waterman local homology algorithm, by the Needleman-Wunsch homology alignment algorithm, by the Pearson-Lipman similarity search method, by computerized implementations of these algorithms or by manual alignment and visual inspection. *See* Smith T, Waterman M., Adv. Appl. Math. 1981; 2:482-489; Needleman S, Wunsch C, J. Mol. Biol. 1970; 48:443-453; Pearson W, Lipman D, Proc. Natl. Acad. Sci. USA 1988; 85:2444-2448; the GAP, BESTFIT, FASTA and TFASTA programs, Wisconsin Genetics Software Package, Genetics Computer Group, Madison, WI, USA; Ausubel F, et al., Eds., "Short Protocols in Molecular Biology", 4th Ed. (John Wiley and Sons, Inc., New York, NY, USA, 1997).

The BLAST and BLAST 2.0 algorithms are suitable for determining percent sequence identity and sequence similarity. *See* Altschul S, et al., Nuc. Acids Res. 1977; 25:3389-3402; Altschul S, et al., J. Mol. Biol. 1990; 215:403-410. The BLAST and BLAST 2.0 programs are used, with the parameters described herein, to determine percent sequence identity for the nucleic acids and proteins of the invention. Software for performing BLAST analyses is publicly available through the National Center for Biotechnology Information (http://blast.ncbi.nlm.nih.gov/blast.cgi, August 2010). This algorithm involves first identifying high scoring sequence pairs (HSPs) through the recognition of short words of length W in the query sequence, which either match or satisfy some positive-valued threshold score T when aligned with a word of the same length in a database sequence. T is referred to as the neighborhood word score threshold. *See* Altschul S, *et al.*, 1997, 1990, *supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative- scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=4 and a comparison of both strands.

The BLAST algorithm also performs a statistical analysis of the similarity between two sequences. *See* Karlin S, Altschul S, Proc. Natl. Acad. Sci. USA 1993; 90:5873-5787. One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

### A polynucleotide encoding growth hormone or a functionally equivalent variant thereof

The term "polynucleotide", as used herein, refers to a polymeric form of nucleotides of any length, either ribonucleotides or deoxyribonucleotides. It also includes modifications, such as methylation or capping and unmodified forms of the polynucleotide. The terms "polynucleotide," "oligomer," "oligonucleotide," and "oligo" are used interchangeably herein. A coding sequence can include, but is not limited to, mRNA, cDNA and recombinant polynucleotide sequences. The polynucleotide that can be used in the therapeutic methods of the invention encodes GH or any of the variants mentioned above. The polynucleotide can be provided as an isolated molecule consisting of the coding sequence for GH or the functionally equivalent variant thereof or may be associated to additional regions which facilitate expression of the GH and or propagation of the polynucleotide.

Typically, the nucleic acid encoding GH or the functionally equivalent variant thereof is provided as a nucleic acid expression construct. The term "nucleic acid expression construct" as used herein refers to any type of an isolated genetic construct comprising a nucleic acid coding for a RNA capable of being transcribed. The term "expression vector" can also be used interchangeably herein. In specific embodiments, the isolated nucleic acid expression construct comprises: a promoter; a nucleotide sequence of interest; and a 3' untranslated region; wherein the promoter, the nucleotide sequence of interest, and the 3' untranslated region are operatively linked and the *in vivo* expression of the nucleotide sequence of interest is regulated by the promoter.

As used herein, a promoter refers to a site in a DNA molecule at which RNA polymerase and transcription factors bind to initiate the transcription of a specific nucleic acid sequence. The phrases "operatively positioned," "operatively linked," "under control" and "under transcriptional control" mean that a promoter is in a correct functional location and/or orientation in relation to a nucleic acid sequence to control transcriptional initiation and/or expression of that sequence. Suitable promoters for use in the present invention include, without limitation, the beta-actin promoter, the muscle creatine kinase promoter, the metallothionein promoter, the albumin promoter, the betaglobin promoter, the insulin promoter, the growth hormone promoter, the troponin I (TN I) promoter, the platelet-derived growth factor promoter and the CMV promoter.

The polynucleotides or expression constructs according to the invention may also be provided as vectors. Vectors suitable for the insertion of said polynucleotide are vectors derived from expression vectors in prokaryotes, such as pUC18, pUC19, Bluescript and derivatives thereof, mp18, mp19, pBR322, pMB9, ColEl, pCR1, RP4, phages and shuttle vectors such as pSA3 and pAT28, expression vectors in yeasts, such as vectors of the type of 2-micron plasmids, integration plasmids, YEP vectors, centromeric plasmids, expression vectors in insect cells, such as vectors of the pAC series and of the pVL series, expression vectors in plants, such as vectors of the pIBI, pEarleyGate, pAVA, pCAMBIA, pGSA, pGWB, pMDC, pMY and pORE series and expression vectors in higher eukaryotic cells based on viral vectors (adenoviruses, adeno-associated viruses as well as retroviruses and, particularly, lentiviruses) as well as non-viral vectors, such as pSilencer 4.1-CMV (Ambion/Applied Biosystems, Foster City, CA, USA), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

### A cell secreting growth hormone or a functionally equivalent variant thereof

The agent capable of increasing the activity of growth hormone can be a cell which is capable of secreting growth hormone or a functionally equivalent variant thereof as defined above. In principle, no limitation exists as to the type of cell that can be used for the purposes of the present invention. By way of illustration, the invention contemplates as suitable host cells mammalian, plant, insect, fungal and bacterial cells. Bacterial cells include, without limitation, Gram-positive bacterial cells, such as species of the *Bacillus*, *Streptomyces and Staphylococcus* genera, and Gram-negative bacterial cells, such as cells of the *Escherichia* and *Pseudomonas* genera. Fungal cells preferably include yeast cells, such as *Saccharomyces*, *Pichia pastoris* and *Hansenula polymorpha*. Insect cells include, without limitation, Drosophila cells and Sf9 cells. Plant cells include, among others, crop plant cells, such as cereal, medicinal, ornamental or bulbous plants. Mammalian cells suitable for the present invention include epithelial cell lines (e.g. porcine), osteosarcoma cell lines (e.g. human), cell lines of neuroblastoma (e.g. human), epithelial carcinomas (e.g. human), glial cells (e.g. murine), liver cell lines (e.g. simian), CHO (chinese hamster ovary) cells, COS cells, BHK cells, HeLa cells, 911 cells, AT1080 cells, A549 cells, 293 cells, PER.C6 cells, human ECC NTERA-2 cells, D3 cells of the mESC line, human embryonic stem cells such as HS293 and BGV01, SHEF1, SHEF2 and HS181, NIH3T3 cells, 293T-cells, REH cells and MCF-7 cells and hMSC cells.

### Effects of the agent capable of increasing the activity of growth hormone in a subject

The expression "increasing the immune response against an immunogenic protein in a subject", as used herein, is understood as a method which allows increasing the immune function in a subject in response to the challenging of the subject with the antigen. The term "improvement" or "increase" are relative (e.g. as compared to a control). Selection of appropriate controls is well within the skill in the art and depends on the particular patient circumstances or purpose of the investigation. For example, in some embodiments, an improvement in the measured immune function is observed relative to that immune function prior to treatment in the same individual. In some embodiments, the improvement is observed compared to a different, but similar individual or group of similar individuals. In some embodiments, the improvement is observed compared to an average value or level for the particular immune function being measured gathered from a population of individuals. The increase in the immune response may prevent or reduce the severity of the infection targeted by the vaccine, may improve the immune response of the individual to the vaccine antigen, even if the infection is not entirely prevented. Again, the terms "improve" and "increase" are relative, and well understood in the art. For example, the agent of the invention can improve the efficacy of a vaccine relative to the efficacy of the same or related vaccine administered to the individual in the absence of the composition. In some embodiments, the improved efficacy is determined relative to an average efficacy gathered from a population of similar individuals (e.g. in the same age range).

According to the present invention, the general use herein of the term "antigen" refers to any portion of a protein (e.g. peptide, partial protein, full-length protein), wherein the protein is naturally occurring or synthetically derived, to a cellular composition (e.g. whole cell, cell lysate, disrupted cells), to an organism (e.g. whole organism, lysate, disrupted cells), to a carbohydrate (e.g. such as those expressed on cancer cells), to other molecule or a portion thereof. An antigen elicits an antigen-specific immune response (e.g. a humoral and/or a cell-mediated immune response) against said antigen when it is administered to a subject. When referring to stimulation of an immune response, the term "antigen" can be used interchangeably with the term "immunogen".

In a preferred embodiment, the method of the invention for increasing the immune response against an antigen in a subject comprises the administration of a vaccine composition comprising said antigen.

The term "vaccine", as used herein, refers to a composition which, upon administration to an immunocompetent subject, elicits an immune response in said subject and which protects the immunized subject against subsequent challenge by the immunizing agent or an immunologically cross-reactive agent.

A variety of adjuvants known to one of ordinary skill in the art may be administered in conjunction with the immunogenic agents in the provided immunological composition. Such adjuvants include but are not limited to the following: polymers; co-polymers such as polyoxyethylene-polyoxypropylene copolymers, including block co-polymers; polymer P1 005; Freund's complete adjuvant (for animals); Freund's incomplete adjuvant; sorbitan monooleate; squalene; CRL-8300 adjuvant; alum; QS 21, muramyl dipeptide; CpG oligonucleotide motifs and combinations of CpG oligonucleotide motifs; trehalose; bacterial extracts, including mycobacterial extracts; detoxified endotoxins; membrane lipids or combinations thereof.

The increase in the immune response attained with the method of the invention can be an increase in the humoral immune response, an increase in the cellular immune response or in both types of response.

The term "humoral response", as used herein, refers to an immune response against foreign antigen(s) that is mediated by antibodies produced by B-cells. The term "humoral response" is used to describe an immune response against foreign antigen(s) that is mediated by antibodies produced by B-cells. A "primary humoral response" results from the activation of naïve lymphocytes (B-cells). A primary response to antigen is generally characterized by a lag time, which is the period of time from antigen encounter until the production of plasma cells and memory cells. The expression "recall response" or "memory response" refer to the immune response to subsequent administration of an antigen.

The term "cellular immune response", which may be used interchangeably with "cell-mediated immune response" refers generally to the response to an antigen of immune cells including T lymphocytes (including cytotoxic T lymphocytes (CTL)), dendritic cells, macrophages, and natural killer cells and to all of the processes that accompany such responses, including, but not limited to, activation and proliferation of these cells, CTL effector functions and cytokine production that influences the function of other cells involved in adaptive immune responses and innate immune responses and memory T-cell generation.

In a preferred embodiment, the therapeutic use of the invention is applied to an immunocompromised subject.

The terms "immunocompromised", "immunodeficient" and "immunosuppressed" are used herein interchangeably to refer to a subject with an innate, acquired, or induced inability to develop a normal immune response. Immunocompromised individuals thus include those with primary (e.g. hereditary) and acquired immunodeficiencies. Immunocompromised subjects include, without limitation, subjects suffering from most forms of cancer (other than skin cancer), subjects which have received chemotherapy or other immunosuppressing treatment, such as induced by treatment with steroids, cyclophosphamide, azathioprine, methotrexate, cyclosporine or rapamycin, in particular in relation to cancer treatment or the treatment or prevention of transplant rejection, subjects suffering from different diseases such as AIDS or leukemia, in particular neutropenia or other secondary immunodeficiencies, an immunodeficiency due to some pathogenic infections or malnutrition, subjects suffering sickle cell anemia, subjects suffering cystic fibrosis, subjects who do not have a spleen, subjects with end stage kidney disease (dialysis), subjects who have been under treatment with corticosteroids, subjects who have received ionizing radiation of various types and strength (e.g. photon, electron, helium nucleus). In a preferred embodiment, the immuncompromised subject suffers an HIV infection or has received a chemotherapy treatment.

In a preferred embodiment, the therapeutic uses of the invention are applied to subjects who do not respond to vaccination with the antigen. The term "subjects who do not respond to vaccination with the immunogenic protein" as used herein, refers to subjects with a proven record of failure to respond to one or more challenges with the antigen. Said failure measured as the inability of the antigen in the subject to elicit a cellular immune response or a humoral immune response.

The ability of a subject to respond to vaccination with an antigen can be measured by any standard method used for determining whether a given antigen is capable of stimulating a cell-mediated or a humoral response.

Thus, the ability of a particular antigen to stimulate a cell-mediated immunological response may be determined by a number of assays, such as by lymphoproliferation (lymphocyte activation) assays, CTL cytotoxic cell assays or by assaying for T-lymphocytes specific for the antigen in a sensitized subject. Such assays are well known in the art. *See* Erickson A, et al. J. Immunol. 1993; 151:4189-4199; Doe B, et al., Eur. J. Immunol. 1994; 24:2369-2376. Recent methods of measuring cell-mediated immune response include measurement of intracellular cytokines or cytokine secretion by T-cell populations, or by measurement of epitope specific T-cells (e.g. tetramer technique). *See* McMichael A, O'Callaghan C, J. Exp. Med. 1998; 187(9):1367-1371; Mcheyzer-Williams M, et al., Immunol. Rev. 1996; 150:5-21; Lalvani A, et al., J. Exp. Med. 1997; 186:859-865.

The ability of a particular antigen to stimulate a B-cell response can be measured by determining the presence of antibodies that bind to the antigen. In one example, neutralizing antibodies are produced. A protective immune response can be measured, for example, by the inhibition of viral replication or plaque formation in a plaque reduction assay or ELISA-neutralization assay (NELISA) or by measuring resistance to viral challenge *in vivo*.

### Administration of the agent capable of increasing the activity of growth hormone

Schedules and dosages for the administration of the agent according to the present invention can be determined in accordance with known methods for these products, for example using the manufacturers' instructions. In a preferred embodiment, the agent according to the present invention is chronically administered.

The expression "chronic administration" refers to administration of the agent(s) in a continuous mode as opposed to an acute mode, each administration occurring before the previous is completely cleared from the subject, so as to maintain the initial therapeutic effect (activity) for an extended period of time. The agent capable of increasing the activity of growth hormone can be administered for a period of time ranging from at least 1 week, at least 2 weeks, at least 3 weeks, at least 1 month, at least 2 months, at least 3 months, at least 4 months, at least 5 months, at least 6 months, at least 7 months, at least 8 months, at least 9 months, at least 10 months and more. Moreover, exemplary dosing schedules include, without limitation, administration five times a day, four times a day, three times a day, twice daily, once daily, three times weekly, twice weekly, once weekly, twice monthly, once monthly and any combination thereof.

Growth hormone induces the secretion of the insulin like growth factor-1 (IGF-1). IGF-1 is closely associated to the levels of GH and regulates thymic homing of T-cells precursors, but is also responsible for the side effects produced by the hormone such as carpian tunnel syndrome, acromegaly and mialgias. Thus, the levels of IGF-1 serve as an indicator of the effect of the therapy according to the invention but also need to be carefully monitored during the trial in order to avoid an excessive increase in the IGF-1 level. In a preferred embodiment, the agent capable of increasing the activity of growth hormone is administered at a dosage that results in IGF-1 serum levels of equal or lower than 500 U/ml. Methods for determining the IGF-1 serum levels are well-known in the art and include, without limitation, RIA assays (e.g. Esoterix RIA kit, (Esoterix, Austin, TX, USA; DSL-2900 Radioimmunoassay Kit (Diagnostic Systems Laboratories, Brea, CA, US), ELISA or by double antibody IGFBP-blocked RIA. *See* Rory F, Siegel N, WO2006/024953; Blum W, Breier B, Growth Regul. 1994; Suppl. 1, 4:11-19.

Suitable dosage regimes for the agent capable of increasing the activity of growth hormone can be determined in a routine manner by the skilled practitioner based on body weight or surface area, but the exact amount used and the frequency of administration may vary between different protocols. For example, exemplary doses of recombinant and native GH polypeptides can be used as a starting point to determine appropriate dosages of the agent. For instance, modified GH polypeptides that are more stable and have an increased half-life *in vivo* can be effective at reduced dosage amounts and or frequencies For example, because of the improvement in properties, such as serum stability, dosages can be lower than comparable amounts of unmodified GH. Dosages for unmodified GH can be used as guidance for determining dosages for modified GH. These doses can be used along with comparisons of properties of the modified and unmodified GH polypeptides to determine dosages for the modified GH The length and dosage of GH treatment can vary according to subject tolerance. Guidance of dosages and dosing from unmodified GH and comparison of properties and activities of modified GH with unmodified GH can be used in the determination. *See* Kotzmann H, et al., Calcif Tissue Intl. 1997; 62(1):40-46.

In a preferred embodiment, the agent capable of increasing the activity of growth hormone is recombinant growth hormone (rGH) and is administered in a dosis of 3 ng three times a week. However, the dose was can be adjusted according to the weight of the patient.

In those cases wherein the agent capable of increasing the activity of GH is a polynucleotide, the administration of the agent is usually carried out using recombinant vectors. The vector can be designed to remain episomal, such as by inclusion of an origin of replication or can be designed to integrate into a chromosome in the cell.

Suitable methods for nucleic acid delivery for transformation of an organelle, a cell, a tissue or an organism for use with the current invention are believed to include virtually any method by which a nucleic acid (e.g. DNA) can be introduced into an organelle, a cell, a tissue or an organism, as described herein or as would be known to one of ordinary skill in the art. Such methods include, but are not limited to, direct delivery of DNA such as by *ex vivo* transfection, by injection including microinjection, by electroporation, by calcium phosphate precipitation, by using DEAE-dextran followed by polyethylene glycol, by direct sonic loading, by liposome mediated transfection and receptor-mediated transfection, by microprojectile bombardment, by agitation with silicon carbide fibers, by Agrobacterium-mediated transformation, by PEG-mediated transformation of protoplasts, by desiccation/inhibition-mediated DNA uptake and any combination of such methods. Through the application of techniques such as these, organelle(s), cell(s), tissue(s) or organism(s) may be stably or transiently transformed.

Human GH polypeptides or functionally equivalent variants thereof can also be used in *ex vivo* gene expression therapy using non-viral vectors. For example, cells can be engineered to express an hGH polypeptide, such as by integrating an hGH polypeptide encoding-nucleic acid into a genomic location, either operatively linked to regulatory sequences or such that it is placed operatively linked to regulatory sequences in a genomic location. Such cells then can be administered locally or systemically to a subject, such as a patient in need of treatment.

Viral vectors, include, for example adenoviruses, herpes viruses, retroviruses and others designed for gene therapy can be employed. The vectors can remain episomal or can integrate into chromosomes of the treated subject. An hGH polypeptide can be expressed by a virus, which is administered to a subject in need of treatment. Virus vectors suitable for gene therapy include adenovirus, adeno-associated virus, retroviruses, lentiviruses and others noted above. For example, adenovirus expression technology is well-known in the art and adenovirus production and administration methods also are well known. Adenovirus serotypes are available, for example, from the American Type Culture Collection (ATCC, Rockville, MD, USA). Adenovirus can be used *ex vivo.* For example, cells are isolated from a patient in need of treatment, and transduced with an hGH polypeptide-expressing adenovirus vector. After a suitable culturing period, the transduced cells are administered to a subject locally and/or systemically. Alternatively, hGH polypeptide-expressing adenovirus particles are isolated and formulated in a pharmaceutically-acceptable carrier for delivery of a therapeutically effective amount to prevent, treat or ameliorate a disease or condition of a subject. Typically, adenovirus particles are delivered at a dose ranging from 1 particle to 10¹⁴ particles per kilogram subject weight, generally between 10⁶ or 10⁸ particles to 10¹² particles per kilogram subject weight. In some situations it is desirable to provide a nucleic acid source with an agent that targets cells, such as an antibody specific for a cell surface membrane protein or a target cell or a ligand for a receptor on a target cell.

The nucleic acid molecules encoding growth hormone or a functionally equivalent variant thereof can be introduced into artificial chromosomes and other non-viral vectors. Artificial chromosomes, such as ACES can be engineered to encode and express the isoform. Briefly, mammalian artificial chromosomes (MACs) provide a means to introduce large payloads of genetic information into the cell in an autonomously replicating, non-integrating format. Unique among MACs, the mammalian satellite DNA-based artificial chromosome expression (ACE), can be reproducibly generated *de novo* in cell lines of different species and be readily purified from the host cells' chromosomes. Purified mammalian ACEs can then be re-introduced into a variety of recipient cell lines where they have been stably maintained for extended periods in the absence of selective pressure using an ACE System. Using this approach, specific loading of one or two gene targets has been achieved in LMTK(-) and CHO cells. In yet another method a two-step gene replacement technique in yeast is utilized. This method encompasses cloning a complete adenovirus genome in a yeast artificial chromosome (YAC) and making a plasmid containing: i) adenovirus sequences to target a specific region in the YAC clone, ii) an expression cassette for the gene of interest and iii) a positive and negative selectable marker. *See* Ketner G, et al., Proc. Natl. Acad. Sci. USA 1994; 91:6186-6190.

The nucleic acids encoding the GH or the functionally equivalent variant thereof can be encapsulated in a vehicle, such as a liposome, or introduced into a cell, such as a bacterial cell, particularly an attenuated bacterium or introduced into a viral vector. For example, when liposomes are employed, proteins that bind to a cell surface membrane protein associated with endocytosis can be used for targeting and/or to facilitate uptake (e.g. capsid proteins or fragments thereof tropic for a particular cell type, antibodies for proteins which undergo internalization in cycling, and proteins that target intracellular localization and enhance intracellular half-life).

For *ex vivo* and *in vivo* methods, nucleic acid molecules encoding the hGH polypeptides are introduced into cells that are from a suitable donor or the subject to be treated. Cells into which a nucleic acid can be introduced for purposes of therapy include, for example, any desired, available cell type appropriate for the disease or condition to be treated, including but not limited to epithelial cells, endothelial cells, keratinocytes, fibroblasts, muscle cells and hepatocytes, blood cells such as T lymphocytes, B lymphocytes, monocytes, macrophages, neutrophils, eosinophils, megakaryocytes and granulocytes and various stem or progenitor cells, in particular hematopoietic stem or progenitor cells (e.g. stem cells obtained from bone marrow, umbilical cord blood, peripheral blood, fetal liver). For *ex vivo* treatment, cells from a donor compatible with the subject to be treated or the subject to be treated cells are removed, the nucleic acid is introduced into these isolated cells and the modified cells are administered to the subject. Treatment includes direct administration, such as, for example, encapsulated within porous membranes, which are implanted into the patient *See* Aebischer P, et al., US 4,892,538; Aebischer P, Wahlberg L, US 5,283,187. Techniques suitable for the transfer of nucleic acid into mammalian cells *in vitro* include the use of liposomes and cationic lipids (e.g. DOTMA, DOPE, DC-Choi) electroporation, microinjection, cell fusion, DEAE-dextran and calcium phosphate precipitation methods. Methods of DNA delivery can be used to express hGH polypeptides *in vivo.* Such methods include liposome delivery of nucleic acids and naked DNA delivery, including local and systemic delivery such as using electroporation, ultrasound and calcium-phosphate delivery. Other techniques include microinjection, cell fusion, chromosome-mediated gene transfer, microcell-mediated gene transfer and spheroplast fusion.

*In vivo* expression of an hGH polypeptide can include operatively linking an hGH polypeptide encoding nucleic acid molecule to specific regulatory sequences such as a cell-specific or tissue-specific promoter. Human GH polypeptides also can be expressed from vectors that specifically infect and/or replicate in target cell types and/or tissues. Inducible promoters can be use to selectively regulate hGH polypeptide expression.

Nucleic acid molecules in the form of naked nucleic acids or in vectors, artificial chromosomes, liposomes and other vehicles can be administered to the subject by systemic administration, topical, local and other routes of administration. When systemic and *in vivo*, the nucleic acid molecule or vehicle containing the nucleic acid molecule can be targeted to a cell.

Administration can also be direct, such as by administration of a vector or cells that typically targets a cell or tissue. For example, tumor cells and proliferating can be targeted cells for *in vivo* expression of hGH polypeptides. Cells used for *in vivo* expression of the hGH polypeptide or the functionally equivalent variant thereof also include cells autologous to the patient. These cells can be removed from a patient, nucleic acids for expression of a hGH polypeptide introduced and then administered to a patient such as by injection or engraftment. Polynucleotides and expression vectors provided herein can be made by any suitable method.

### Antigens for use in the method of the invention

In preferred embodiments, the antigen is an immunogenic protein. The term "immunogenic protein", as used herein, describes a polypeptide which elicits a humoral and/or cell-mediated immune response (i.e. is antigenic).

In a still more preferred embodiment, the immunogenic protein is selected from the group consisting of a cancer antigen, a bacterial antigen, a protozoal antigen, a viral antigen and an allergen.

The antigenic polypeptide, homologous or heterologous, can be, for example, but is not limited to, a viral antigen, a bacterial antigen, a protozoal antigen, a fungal antigen, a differentiation antigen, a tumor antigen, an embryonic antigen, an antigen of oncogenes and mutated tumor-suppressor genes, a unique tumor antigen resulting from chromosomal translocations and/or derivatives thereof.

Viral antigens which are capable of eliciting an immune response against the virus include HIV-1 antigens (e.g. tat, nef, gp120 or gp160, gp40, p24, gag, env, vif, vpr, vpu, rev), human herpes viruses (e.g. gH, gL gM gB gC gK gE or gD or derivatives thereof), or immediate early protein (e.g. ICP27 , ICP47, ICP4, ICP36 from HSV1 or HSV2), cytomegalovirus, especially human (e.g. gB or derivatives thereof), Epstein-Barr virus (e.g. gp350 or derivatives thereof), varicella zoster virus (e.g. gp1, II, I11 and IE63), or from a hepatitis virus like hepatitis B virus (e.g. hepatitis B surface antigen or hepatitis core antigen), hepatitis C virus (e.g. core, E1, NS3 or NS5 antigens), from paramyxoviruses such as respiratory syncytial virus (e.g. F and G proteins or derivatives thereof), from parainfluenza virus, from rubella virus (e.g. proteins E1 and E2), measles virus, mumps virus, human papilloma viruses (e.g. HPV6, 11, 16, 18, eg L1, L2, E1, E2, E3, E4, E5, E6, E7), flaviviruses (e.g. yellow fever virus, dengue virus, tick-borne encephalitis virus, Japanese encephalitis virus) or influenza virus cells (e.g. HA, NP, NA, or M proteins, or combinations thereof) and rotavirus antigens (e.g. VP7sc and other rotaviral components). *See* Murphy F, Kingsbury D, Eds., "Fundamental Virology", 2nd Ed. (Raven Press, New York, NY, USA, 1991). In preferred embodiments, the viral antigen is selected from the group consisting of a hepatitis A antigen, an hepatitis B antigen, an hepatitis C antigen and a HIV-1 antigen.

Bacterial antigens which can be encoded by the polynucleotide of the present invention include antigens from *Neisseria spp,* including *N. gonorrhea* and *N. meningitidis* (transferrin-binding proteins, lactoferrin binding proteins, PiIC and adhesins); antigens from *S. pyogenes* (e.g. M proteins or fragments thereof and C5A protease); antigens from *S. agalactiae, S. mutans*; *H. ducreyi; Moraxella spp,* including *M. catarrhalis*, also known as *Branhamella catarrhalis* (e.g. high and low molecular weight adhesins and invasins); antigens from *Bordetella spp*, including *B. pertussis* (e.g. parapertussis and *B. bronchiseptica* (e.g. pertactin, pertussis toxin or derivatives thereof, filamenteous hemagglutinin, adenylate cyclase, fimbriae); antigens from *Mycobacterium spp*, including *M. tuberculosis, M. bovis*, *M. leprae*, *M. avium*, *M. paratuberculosis*, *M. smegmatis*; *Legionella spp*, including *L. pneumophila*; (e.g. ESAT6, Antigen 85A, -B or -C, MPT 44, MPT59, MPT45, HSPIO,HSP65, HSP70, HSP 75, HSP90, PPD 19kDa [Rv3763], PPD 38kDa [Rv0934] ); antigens from *Escherichia spp*, including enterotoxic *E. coli* (e.g. colonization factors, heat-labile toxin or derivatives thereof, heat-stable toxin or derivatives thereof), antigens from enterohemorragic *E. coli* and enteropathogenic *E. coli* (e.g. shiga toxin-like toxin or derivatives thereof); antigens from *Vibrio spp*, including *V. cholera* (e.g. cholera toxin or derivatives thereof); antigens from *Shigella spp*, including *S. sonnei*, *S. dysenteriae*, *S. flexnerii*; *Yersinia spp*, including *Y. enterocolitica* (e.g. a Yop protein); antigens from *Y. pestis, Y. pseudotuberculosis; Campylobacter spp*, including *C. jejuni* (e.g. toxins, adhesins and invasins); antigens from *Salmonella spp*, including *S. typhi*, *S. paratyphi*, *S. choleraesuis*, *S. enteritidis; Listeria spp*, including *L. monocytogenes; Helicobacter spp*, including *H. pylori* (e.g. urease, catalase, vacuolating toxin); antigens from *Pseudomonas spp,* including *P. aeruginosa; Staphylococcus spp,* including *S. aureus*, *S. epidermidis*; *Enterococcus spp*, including *E. faecalis, E. faecium; Clostridium spp*, including *C. tetani* (e.g. tetanus toxin and derivative thereof); antigens from *C. botulinum* (e.g. botulinum toxin and derivative thereof), antigens from *C. difficile* (e.g. clostridium toxins A or B and derivatives thereof); antigens from *Bacillus spp,* including *B. anthracis* (e.g. anthrax toxin and derivatives thereof); *Corynebacterium spp*, including *C. diphtheriae* (e.g. diphtheria toxin and derivatives thereof); antigens from *Borrelia spp*, including *B. burgdorferi* (e.g. OspA, OspC, DbpA, DbpB); antigens from *B. garinii* (e.g. OspA, OspC, DbpA, DbpB), *B. afzelii* (e.g. OspA, OspC, DbpA, DbpB), antigens from *B. andersonfi* (e.g. OspA, OspC, DbpA, DbpB), antigens from *B. hermsii*; *Ehrlichia spp*, including *E. equi* and the agent of the human granulocytic ehrlichiosis; *Rickettsia spp*, including *R. rickettsii*; *Chlamydia spp*, including *C. trachomatis* (e.g. MOMP, heparin-binding proteins); antigens from *Chlamydia pneumoniae* (e.g. MOMP, heparin-binding proteins), antigens from *C. psittaci*; *Leptospira spp*, including *L. interrogans*; *Treponema spp*, including *T. pallidum* (e.g. the rare outer membrane proteins), antigens from *T. denticola*, *T. hyodysenteriae*; antigens from *Plasmodium spp*, including *P. falciparum*; *Toxoplasma spp* and *T. gondii* (e.g. SAG2, SAGS, Tg34); antigens from *Entamoeba spp*, including *E. histolytica*; *Babesia spp*, including *B. microti; Trypanosoma spp*, including *T. cruzi; Giardia spp*, including *G. lamblia; Leishmania spp*, including *L. major; Pneumocystis spp*, including *P. carinii; Trichomonas spp*, including *T. vaginalis; Schisostoma spp*, including *S. mansoni*, or derived from yeast such as *Candida spp*, including *C. albicans*; *Cryptococcus spp*, including *C. neoformans*; antigens from *M. tuberculosis* (e.g. Rv2557, Rv2558, RPFs: Rv0837c, Rv1884c, Rv2389c, Rv2450, Rv1009, aceA (Rv0467), PstS1, (Rv0932), SodA (Rv3846), Rv2031c 16kDal., Tb Ra12, Tb H9, Tb Ra35, Tb38-1, Erd 14, DPV, MTI, MSL, mTTC2 and hTCCl); antigens from *Chlamydia spp* (e.g. the high molecular weight protein (HWMP), ORF3 (EP 366 412)), and putative membrane proteins (Pmps); antigens from *Streptococcus spp,* including *S. pneumoniae* (PsaA, PspA, streptolysine, choline-binding proteins, the protein antigen Pneumolysin, and mutant detoxified derivatives thereof); antigens derived from *Haemophilus spp*, including *H. influenzae* type B (e.g. PRP and conjugates thereof); antigens from non typeable *H. influenzae* (e.g. OMP26, high molecular weight adhesins, P5, P6, protein D and lipoprotein D, and fimbrin and fimbrin derived peptides, or multiple copy variants or fusion proteins thereof); antigens derived from *Plasmodium falciparum* (e.g. RTS.S, TRAP, MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in *Plasmodium spp*). In a preferred embodiment, the bacterial antigen is the tetanus toxoid.

Fungal antigens which can be encoded by the polynucleotides of the present invention include, without limitation, Candida fungal antigen components; histoplasma fungal antigens, such as heat shock protein 60 (HSP60) and other histoplasma fungal antigen components; cryptococcal fungal antigens, like capsular polysaccharides and other cryptococcal fungal antigen components; coccidiodes fungal antigens, such as spherule antigens, and other coccidiodes fungal antigen components and tinea fungal antigens, like trichophytin and other coccidiodes fungal antigen components.

Protozoal antigens which can be encoded by the polynucleotides of the present invention include, without limitation, *Plasmodium falciparum* antigens (e.g. merozoite surface antigens), sporozoite surface antigens, circumsporozoite antigens, gametocyte/gamete surface antigens, blood-stage antigen pf, 55/RESA and other plasmodial antigen components, toxoplasma antigens (e.g. SAG-I, p30 and other toxoplasmal antigen components), schistosomae antigens (e.g. glutathione-S-transferase, paramyosin and other schistosomal antigen components), leishmania major and other leishmaniae antigens (e.g. gp63, lipophosphoglycan and its associated protein and other leishmanial antigen components) and *Trypanosoma cruzi* antigens (e.g. the 75-77 kDa antigen, the 56 kDa antigen and other trypanosomal antigen components).

Allergens or environmental antigens which can be encoded by the polynucleotides of the present invention include, without limitation, antigens derived from naturally occurring allergens, such as pollen allergens (e.g. tree-, herb, weed- and grass pollen allergens), insect allergens (inhalant, saliva and venom allergens), animal hair and dandruff allergens, and food allergens. Important pollen allergens from trees, grasses and herbs originate from the taxonomic orders of Fagales, Oleales, Pinoles and platanaceae including La. birch (Betula), alder (Alnus), hazel (Corylus), hornbeam (Carpinus) and olive (Olea), cedar (*Cryptomeriaand juniperus*), Plane tree (Platanus), the order of Poales (e.g. grasses of the genera Lolium, Phleum, Poa, Cynodon, Dactylis, Holcus, Phalaris, Secale and Sorghum) the orders of Asterales and Urticales (e.g. herbs of the genera Ambrosia, Artemisia and Parietaria). Other allergen antigens that may be used include allergens from house dust mites of the genus Dermatophagoides and Euroglyphus, storage mite (e.g. Lepidoglyphys, Glycyphagus and Tyrophagus), those from cockroaches, midges and fleas (e.g. Blatella, Periplaneta, Chironomus and Ctenocepphalides), those from mammals (e.g. cat, dog and horse), birds, venom allergens including such originating from stinging or biting insects such as those from the taxonomic order of Hymenoptera including bees (e.g. superfamily Apidae), wasps and ants (e.g. superfamily Formicoidae). Still other allergen antigens that may be used include inhalation allergens from fungi (e.g. from the genera Alternaria and Cladosporium).

Tumor antigens which can be encoded by the polynucleotides of the present invention include, without limitation, MAGE, MART-1/Melan-A, gp100, Dipeptidyl peptidase IV (DPPIV), adenosine deaminase-binding protein (ADAbp), cyclophilin b, Colorectal associated antigen (CRC)-0017-1A/GA733, Carcinoembryonic Antigen (CEA) and its antigenic epitopes CAP-1 and CAP-2, etv6, aml1, Prostate Specific Antigen (PSA) and its antigenic epitopes PSA-1, PSA-2, and PSA-3, prostate-specific membrane antigen (PSMA), T-cell receptor/CD3-ς chain, MAGE-family of tumor antigens (e.g. MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g. GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p2lras, RAS1, α-fetoprotein, E-cadherin, α-catenin,13-catenin, γ-catenin, p12Octn, gp100Pme1117, PRAME, NY-ESO-1, cdc27, adenomatous polyposis coli protein (APC), fodrin, Connexin 37, Ig- idiotype, p15, gp75, GM2 and GD2 gangliosides, viral products, like human papilloma virus proteins, Smad family of tumor antigens, lmp-1, P1A, EBV-encoded nuclear antigen (EBNA)-1, brain glycogen phosphorylase, SSX-1, SSX-2 (HOM-MEL-40), SSX-3, SSX-4, SSX-5, SCP-1 and CT-7, and c-erbB-2, acute lymphoblastic leukemia (etv6, amll, cyclophilin b), B-cell lymphoma (Ig-idiotype), glioma (E-cadherin, α-catenin,13-catenin, 7-catenin, p120ctn), bladder cancer (p2lras), biliary cancer (p2lras), breast cancer (MUC family, HER2/neu, c-erbB-2), cervical carcinoma (p53, p2lras), colon carcinoma (p2lras, HER2/neu, c-erbB-2, MUC family), colorectal cancer (Colorectal associated antigen (CRC)-0017-1A/GA733, APC), choriocarcinoma (CEA), epithelial cell cancer (cyclophilin b), gastric cancer (HER2/neu, c-erbB-2, ga733 glycoprotein), hepatocellular cancer, Hodgkins lymphoma (lmp-1, EBNA-1), lung cancer (CEA, MAGE-3, NY-ESO-1), lymphoid cell-derived leukemia (cyclophilin b), melanoma (p15 protein, gp75, oncofetal antigen, GM2 and GD2 gangliosides, Melan-A/MART-1, cdc27, MAGE-3, p2lras, gp100Pme1117), myeloma (MUC family, p2lras), non-small cell lung carcinoma (HER2/neu, c-erbB-2), nasopharyngeal cancer (lmp-1, EBNA-1), ovarian cancer (MUC family, HER2/neu, c-erbB-2), prostate cancer (Prostate Specific Antigen (PSA) and its antigenic epitopes PSA-1, PSA-2, and PSA-3, PSMA, HER2/neu, c-erbB-2, ga733 glycoprotein), renal cancer (HER2/neu, c-erbB-2), squamous cell cancers of the cervix and esophagus (viral products e.g. human papilloma virus proteins), testicular cancer (NY-ES0-1) and T-cell leukemia (HTLV-1 epitopes).

### Administration of the antigen according to the methods of the invention

The relative order of administration of the agent capable of increasing the activity of growth hormone and of the antigen is not a limiting aspect of the present invention as long as an increase in the immune response to the later is elicited. Thus, the invention contemplates methods wherein the agent capable of increasing the activity of growth hormone and the antigen or the vaccine composition comprising the agent are administered simultaneously, sequentially or separately.

The term "simultaneous administration," as used herein, means that the agent capable of increasing the activity of growth hormone and the antigen are administered with a time separation of no more than about 15 minute(s), such as no more than about any of 10, 5 or 1 minutes.

The term "separated administration" encompasses administering the agent capable of increasing the activity of growth hormone and the antigen at different times, in any order, whether overlapping or not. This includes, but is not limited to, sequential treatment (such as pretreatment, post-treatment, or overlapping treatment) with the components of the combination, as well as regimens in which the drugs are alternated, or wherein one component is administered long- term and the other(s) are administered intermittently. Components may be administered in the same or in separate compositions, and by the same or different routes of administration.

In a preferred embodiment, the agent capable of increasing the activity of growth hormone and the antigen are administered separately. In another preferred embodiment, the agent capable of increasing the activity of growth hormone is administered after the antigen. In a still more preferred embodiment, the agent capable of increasing the activity of growth hormone is administered chronically and the antigen is administered at least once during the chronic administration of the agent. In a more preferred embodiment, the agent capable of increasing the activity of growth hormone is administered for at least 1, 2, 3, 4, 5, 6, 7, 8, 9, 10, 11 or 12 months and the antigen is administered at week 1, at week 2, at week 3, at week 4, at week 8, at week 12, at week 16, at week 20 and/or at week 24. In a more preferred embodiment, the agent capable of increasing the activity of growth hormone is administered for 6 months and the antigen is administered at week 16.

The agent capable of increasing the activity of growth hormone and the antigen can be administered by the same or by a different route. Therefore, suitable routes of administration of the agent capable of increasing the activity of growth hormone include, without limitation, topical, oral, aerosol, intranasal, intraarterial, intradermal, intravesical, intraocular, parenteral, subcutaneous, intrathecal, intravenous, intramuscular, interperitoneal, rectal, vaginal or transdermal administration. Similarly, suitable routes of administration of the antigen include, without limitation, topical, oral, aerosol, intranasal, intraarterial, intradermal, intravesical, intraocular, parenteral, subcutaneous, intrathecal, intravenous, intramuscular, interperitoneal, rectal, vaginal or transdermal administration.

The volume of administration of the antigen will vary depending on the route of administration. By way of example, intramuscular injections may range from about 0.1 ml to 1.0 ml. Those of ordinary skill in the art will know appropriate volumes for different routes of administration. The amount of antigen in each immunological composition dose is selected as an amount that induces an immunoprotective response in immunocompetent subjects without significant, adverse side effects. Such amount will vary depending upon which specific immunogen is employed and how it is presented. Doses for human administration of a pharmaceutical composition or a vaccine may be from about 0.01 mg/kg to 10 mg/kg, for instance approximately 1 mg/kg. Based on this range, equivalent dosages for heavier (or lighter) body weights can be determined. The dose may be adjusted to suit the individual to whom the composition is administered, and may vary with age, weight, and metabolism of the individual, as well as the health of the subject. Such determinations are left to the attending physician or another familiar with the subject and/or the specific situation. Following an initial vaccination, subjects may receive one or several booster immunizations, adequately spaced. Booster injections may range from 1 µg to 1 mg, with other embodiments having a range of approximately 10 µg to 750 µg, and still others a range of about 50 µg to 500 µg. Periodic boosters at intervals of 1-5 years, for instance three years, may be desirable to maintain the desired levels of protective immunity.

The antigen or vaccine composition comprising the agent is packaged in a single dosage for immunization by parenteral (e.g. intramuscular, intradermal, subcutaneous) administration or nasopharyngeal (e.g. intranasal) administration. In certain embodiments, the immunological composition is injected intramuscularly into the deltoid muscle. The immunological composition may be combined with a pharmaceutically acceptable carrier to facilitate administration. The carrier is, for instance, water, or a buffered saline solution, with or without a preservative. The immunological composition may be lyophilized for resuspension at the time of administration or in solution.

In a preferred embodiment, the agent capable of increasing the activity of growth hormone is administered intramuscularly.

### Compositions of the invention

The invention also provides compositions and kits, hereinafter compositions or kits of the invention, which comprise an agent capable of increasing the activity of growth hormone and an antigen. Thus, in another aspect, the invention relates to a composition or kit comprising:
(i) an agent capable of increasing the activity of growth hormone and
(ii) an antigen in amounts sufficient to induce an immune response against said antigen.

The terms "agent capable of increasing the activity of growth hormone", "growth hormone", "functionally equivalent variant of growth hormone" and "antigen" have been described in detail above and are equally applicable to the compositions of the invention. The term "in amounts sufficient to induce an immune response" when referred to the antigen composition indicates that the antigen is present in amounts or formulation suitable for inducing an immune response in an immunocompetent subject. The term "immunocompetent subject" refers to a mammal having an immune system that, upon exposure to an immunogenic epitope, is capable of mounting a cellular and/or humoral immune response against the immunogenic epitope.

In a preferred embodiment, the agent capable of increasing the activity of growth hormone is selected from the group consisting of:
(i) growth hormone or a functionally equivalent variant thereof,
(ii) a polynucleotide encoding growth hormone or a functionally equivalent variant thereof and
(iii) a cell secreting growth hormone or a functionally equivalent variant thereof.

In a still more preferred embodiment, the agent capable of increasing the activity of growth hormone is not a growth hormone secretagogue as described hereinabove.

In a preferred embodiment, the immunogenic protein is selected from the group consisting of a cancer antigen, a bacterial antigen, a protozoal antigen, a viral antigen and an allergen. In a still more preferred embodiment, the immunogenic protein is a viral antigen. In yet another preferred embodiment, the viral antigen is selected from the group consisting of a hepatitis A antigen, a hepatitis B antigen and a HIV-1 antigen.

In a still more preferred embodiment, the immunogenic protein is a bacterial antigen. In yet another preferred embodiment, the bacterial antigen is the tetanus toxoid.

In another embodiment, the invention relates to a composition or kit according to the invention for use in medicine.

In another embodiment, the invention relates to a composition or kit according to the invention for use as a vaccine. Alternatively, the invention relates to the use of a composition or kit according to the invention for the manufacture of a vaccine composition. Alternatively, the invention relates to a method for inducing an immune response against an antigen in a subject in need thereof which comprises the administration of the composition or kit according to the invention to said subject.

Diseases than can be treated or prevented using the compositions or kits of the invention include any disease which requires an immune response against a given antigen and includes both opportunistic and non-opportunistic infections, where the term "opportunistic" as used herein denotes those infections caused by organisms capable of causing a disease only in a host whose resistance is lowered (e.g. by chemotherapy or HIV infection).

As described herein, administration of an agent capable of increasing the activity of growth hormone in conjunction with exposure to an antigen or administration of a vaccine enhances antigen-specific immune responses. The antigen can be any type of antigen against which an immune response is desired in a subject, or any antigen to which a subject is exposed. In some cases, a subject is exposed to the antigen during an infection, such as a viral, bacterial, fungal or parasitic infection. In other cases, the subject has a tumor and is exposed to a tumor-specific antigen. Alternatively, the antigen can be administered to a subject, such as in the form of a vaccine. In some embodiments, the vaccine is a vaccine against a pathogen, or a cancer vaccine.

In some embodiments, the antigen is an antigen from a pathogen, such as a virus, bacterium, fungus or parasite. Viral pathogens include, but are not limited to retroviruses, such as human immunodeficiency virus (HIV) and human T-cell leukemia viruses; picornaviruses, such as polio virus, hepatitis A virus; hepatitis C virus, enteroviruses, human coxsackie viruses, rhinoviruses, echoviruses, and foot- and-mouth disease virus; calici viruses, such as strains that cause gastroenteritis (e.g. Norwalk virus); togaviruses, such as alphaviruses (including chikungunya virus, equine encephalitis viruses, Sindbis virus, Semliki Forest virus, and Ross River virus) and rubella virus; flaviviruses, such as dengue viruses, yellow fever viruses, West Nile virus, St. Louis encephalitis virus, Japanese encephalitis virus, Powassan virus and other encephalitis viruses; coronaviruses, including severe acute respiratory syndrome (SARS) virus; rhabdoviruses, such as vesicular stomatitis virus and rabies virus; filoviridae viruses, such as Ebola virus and Marburg virus; paramyxoviruses, such as parainfluenza virus, mumps virus, measles virus, and respiratory syncytial virus; orthomyxoviruses, such as influenza viruses (including avian influenza viruses and swine influenza viruses); bunyaviruses, such as Hantaan virus; Sin Nombre virus, and Rift Valley fever virus, phleboviruses and Nairo viruses; arenaviruses, such as Lassa fever virus and other hemorrhagic fever viruses, Machupo virus and Junin virus; reoviruses, such as mammalian reoviruses, orbiviurses and rotaviruses; birnaviruses; hepadnaviruses, such as hepatitis B virus; parvoviruses; papovaviruses, such as papilloma viruses, polyoma viruses and BK- virus; adenoviruses; herpesviruses, such as herpes simplex virus (HSV)-I and HSV- 2, cytomegalovirus, Epstein-Barr virus, varicella zoster virus, and other herpes viruses, including HSV-6); pox viruses, such as variola viruses and vaccinia viruses; irodoviruses, such as African swine fever virus; astroviruses and unclassified viruses (e.g. the etiological agents of spongiform encephalopathies; the agent of delta hepatitis (thought to be a defective satellite of hepatitis B virus)).

Bacterial pathogens include, but are not limited to *Helicobacter pylori*, *Escherichia coli, Vibrio cholerae*, *Borelia burgdorferi*, *Legionella pneumophilia, Mycobacteria spp* (e.g. *M. tuberculosis*, *M. avium*, *M. intracellular*, *M. kansai*, *M. gordonae*), *Staphylococcus aureus*, *Neisseria gonorrhoeae, Neisseria meningitidis, Listeria monocytogenes*, *Streptococcus pyogenes* (Group A streptococcus), *Streptococcus agalactiae* (Group B streptococcus), Streptococcus (viridans group), *Streptococcus faecalis, Streptococcus bovis,* Streptococcus (anaerobic spp), *Streptococcus pneumoniae*, pathogenic *Campylobacter spp*, *Enterococcus spp*, *Haemophilus influenzae*, *Bacillus anthracis, Corynebacterium diphtheriae*, *Corynebacterium spp*, *Erysipelothrix rhusiopathiae, Clostridium perfringers, Clostridium tetani, Enterobacter aerogenes, Klebsiella pneumoniae, Pasturella multocida, Bacteroides spp, Fusobacterium nucleatum, Streptobacillus moniliformis*, *Treponema pallidium, Treponema pertenue, Leptospira spp, Bordetella pertussis, Shigella flexnerii*, *Shigella dysenteriae* and *Actinomyces israelii.*

Fungal pathogens include, but are not limited to *Cryptococcus neoformans*, *Histoplasma capsulatum, Coccidioides immitis, Blastomyces dermatitidis, Chlamydia trachomatis*, *Candida albicans.* Parasitic pathogens include, but are not limited to *Plasmodium falciparum*, *Plasmodium vivax*, *Trypanosoma cruzi* and *Toxoplasma gondii.*

In some cases, the antigen is a tumor-associated antigen. Tumor antigens are proteins that are produced by tumor cells that elicit an immune response, particularly T-cell mediated immune responses. The tumor antigen can be any tumor-associated antigen, which are well known in the art and include, for example, carcinoembryonic antigen (CEA), beta -human chorionic gonadotropin, alphafetoprotein (AFP), lectin-reactive AFP, thyroglobulin, RAGE-I, MN-CA IX, human telomerase reverse transcriptase, RU1, RU2 (AS), intestinal carboxyl esterase, mut hsp70-2, macrophage colony stimulating factor, prostase, prostate-specific antigen (PSA), PAP, NY-ESO-I, LAGE-Ia, p53, prostein, PSMA, Her2/neu, survivin and telomerase, prostate-carcinoma tumor antigen- 1, MAGE, ELF2M, neutrophil elastase, ephrinB2, CD22, insulin growth factor (IGF)-I, IGF-II, IGF-I receptor and mesothelin.

The compositions and kits of the invention may be used to increase the immune response against any vaccine used in the art. In particular, suitable vaccines for use in the present invention include, without limitation, adsorbed anthrax vaccine (BIOTHRAX); BCG vaccine (TICE BCG and MYCOBAX); adsorbed diphtheria and tetanus vaccine; adsorbed diphtheria, tetanus and acellular pertussis vaccine (TRIPEDIA, INFANRIX, DAPTACEL); diphtheria, tetanus and acellular pertussis (PEDIARIX); adsorbed hepatitis b (recombinant) and inactivated poliovirus combined vaccine; adsorbed diphtheria, tetanus and acellular pertussis and inactivated poliovirus vaccine (KINRIX); adsorbed inactivated poliovirus and haemophilus b conjugate (tetanus toxoid conjugate) vaccine (PENTACEL); haemophilus b conjugate vaccine (diphtheria; CRM197 protein conjugate) (HIBTITER); haemophilus b conjugate vaccine (meningococcal; protein conjugate) (PEDVAXHIB); haemophilus b conjugate vaccine (tetanus toxoid conjugate) (ACTHIB); haemophilus b conjugate vaccine (meningococcal protein conjugate) and hepatitis B vaccine (recombinant) (COMVAX); inactivated hepatitis A vaccine (HAVRIX); inactivated hepatitis A vaccine (VAQTA); inactivated hepatitis A and hepatitis B (recombinant) vaccine (TWINRIX); hepatitis B vaccine (recombinant) (RECOMBIVAX HB); hepatitis B vaccine (recombinant) (ENGERIX-B); human papillomavirus (types 6, 11, 16, 18) recombinant vaccine (GARDASIL); influenza virus vaccine (AFLURIA); influenza H5N1 virus vaccine; influenza trivalent virus types a and b vaccine (FLULAVAL); live intranasal influenza virus vaccine (FLUMIST); influenza trivalent types a and b virus vaccine (FLUARIX); influenza trivalent types a and b virus vaccine (FLUVIRIN); influenza trivalent types a and b virus vaccine (FLUZONE); inactivated Japanese encephalitis virus vaccine (JE-VAX); live measles virus vaccine (ATTENUVAX); live measles and mumps virus vaccine (M-M-Vax); live measles, mumps and rubella virus vaccine (M-M-R II); live measles, mumps, rubella and varicella virus vaccine (PROQUAD); meningococcal polysaccharide (serogroups A, C, Y and W-135) and diphtheria conjugate vaccine (MENACTRA); meningococcal polysaccharide combined vaccine (serogroups A, C, Y and W-135) (MENOMUNE-A/C/Y/W); live mumps virus vaccine (MUMPSVAX); plague vaccine; pneumococcal polyvalent vaccine (PNEUMOVAX 23); pneumococcal 7-valent conjugate vaccine (diphtheria CRM197 protein) (PREVNAR); inactivated poliovirus vaccine (human diploid cell) (POLIOVAX); inactivated poliovirus vaccine (monkey kidney cell) (IPOL); rabies vaccine (IMOVAX); rabies vaccine (RABAVERT); adsorbed rabies vaccine; live oral rotavirus vaccine (ROTARIX); live oral pentavalent rotavirus vaccine (ROTATEQ); live rubella virus vaccine (MERUVAX II); live smallpox (vaccinia) vaccine (ACAM2000); dried calf lymph type smallpox vaccine (DRYVAX); adsorbed diphtheria and tetanus vaccine for adult use; adsorbed diphtheria and tetanus vaccine for adult use (DECAVAC); adsorbed diphtheria and tetanus vaccine for adult use (TENIVAC); adsorbed diphtheria and tetanus vaccine (DITANRIX); tetanus vaccine; adsorbed tetanus vaccine; adsorbed reduced diphtheria, tetanus and acellular pertussis vaccine (ADACEL); adsorbed reduced diphtheria, tetanus and acellular pertussis vaccine (BOOSTRIX); live oral ty2la typhoid vaccine (VIVOTIF); typhoid VI polysaccharide vaccine (TYPHIM VI); live varicella virus vaccine (VARIVAX); yellow fever vaccine (YF-VAX) and live varicella zoster vaccine (ZOSTAVAX).

In another embodiment, the composition according the invention contains an agent that is not a growth hormone secretagogue. Preferably, the agent is selected from the group consisting of:
(i) growth hormone or a functionally equivalent variant thereof,
(ii) a polynucleotide encoding growth hormone or a functionally equivalent variant thereof and
(iii) a cell secreting growth hormone or a functionally equivalent variant thereof.

In yet another embodiment, the composition or kit according to the invention contains an antigen selected from the group consisting of a cancer antigen, a bacterial antigen, a viral antigen and an allergen. Preferably, the antigen is a viral antigen. More preferably, the viral antigen is selected from the group consisting of hepatitis A virus antigen, a hepatitis C antigen and a HIV-1 antigen.

In another embodiment, the composition or kit according to the invention contains a bacterial antigen. Preferably, the bacterial antigen is the tetanus toxoid.

In a further embodiment, the composition or kit according to the invention is for use in medicine. Preferably, it is for use as vaccine.

In an additional embodiment, the agent and antigen of the composition or kit according to the invention are administered simultaneously, sequentially or separately.

All publications mentioned hereinabove are hereby incorporated in their entirety by reference.

While the foregoing invention has been described in some detail for purposes of clarity and understanding, it will be appreciated by one skilled in the art from a reading of this disclosure that various changes in form and detail can be made without departing from the true scope of the invention and appended claims.

The examples below are intended to further illustrate certain preferred embodiments of the invention and are not intended to limit the scope of the invention.

### EXAMPLES

### General Procedures

### 1. Inclusion criteria

Prospects were tested for HIV, hepatitis A, hepatitis B and tetanus toxoid. The patients that were negative for any of these antigens were selected for revaccination. Those that still showed negative antibody activity after vaccination entered the study. The inclusion criteria were: HIV positive patients, ≥18 years of age, undergoing HAART for at least one year before the beginning of the trial, Karnofsky performance status scale ≥80%, to have undetectable HIV plasma load (< 50 copies per mL) and more than 50 CD4 cells per mm3. To have AST and ALT serum values ≤ 5 times the normal superior limit (NSL), bilirubin in serum ≤ 2 times NSL, granulocytes counts > 1000 cells per mm3, hemoglobin ≥9.5 g per dL and platelets ≥75000 per mm3. No HIV-wasting or current acquired immune deficiency syndrome (AIDS)-defining diseases, and no other serious chronic diseases or cancer, diabetes mellitus, allergy or hyperreactivity to recombinant growth hormone or vaccines, drug or alcohol abuse or therapy with systemic glucocorticoids, as well as non pregnancy or maternity.

Eligible HIV-infected patients were randomized in three different arms: Group A received recombinant growth hormone during 6 months and were vaccinated with Hepatitis A+B (Twinrix Adults®, GlaxoSmithKline, Middlesex, UK) or tetanus toxoid (Ditanrix Adult®, GlaxoSmithKline, Middlesex, UK) at week 16; Group B received recombinant growth hormone but were not vaccinated; and Group C (HIV control group, received vaccines at week 16 but not recombinant growth hormone. The rGH utilized in the trial was GENOTONORM® Kapiben 12 mg (Pfizer, New York, NY, USA). The rGH was injected intramuscularly in the quadriceps, preferentially in the morning for 6 months. All HIV positive individuals received HAART for at least one year before commencing the trial and during its duration.

The dose of rGH recommended in the trial was 3ng three times a week, but the dose was adjusted according to the weight of the patient. Growth hormone induces the secretion of the insulin like growth factor-1 (IGF-1). IGF-1 is closely associated to the levels of GH and regulates thymic homing of T-cells precursors, but is also responsible for the side effects produced by the hormone such as carpian tunnel syndrome, acromegaly and mialgias. Thus, the levels of IGF-1 were carefully monitored during the trial and the doses of rGH doses were reduced until the level of IGF-1 was within the normal range (<500U/ml). Chest computerized tomograms (CT) were performed on the patients to evaluate the thymus.

### 2. Measurement of T-cell receptor excision circle (TRECS)

Peripheral blood mononuclear cells (PBMCs) were frozen in a pellet at -80°C until DNA extraction. Genomic DNA was extracted by using QIAamp DNA Blood Mini Kit (QIAgen, Valencia, CA, USA) according to the manufacturer's instructions. The number of TRECs and CCR5 was measured by real time quantitative PCR that was performed in a spectrofluorometric thermal cycler (ABI PRISM 7000; Applied Biosystems, Foster City, CA, USA) as previously described. *See* Ruiz-Hernández R, et al., The Open Immunol. J. 2010; 3(8):19-26. The number of TRECs was related to the number of CCR5 copies in the same DNA samples.

### 3. Immunophenotyping

T-cell subpopulations were detected in fresh whole blood by a direct immunofluorescence method using four fluorochrome combinations of reagents and analyzed using a Facscalibur cytometer (Becton Dickinson, Cowley, Oxford, UK). *See* Bofill M, et al., Eur. Re. J. 1998; 11(3):548-553. EDTA treated peripheral blood was labeled with different combinations of antibodies: 1) with CD3, CD19, CD14 and CD56 for the detection ofmonocytes, T, B and natural killer cells; 2) with CD4, CD56RA and CD31 for the detection of recent thymic emigrants; 3) with CD62L, CD45RA and CD45RO for naïve and memory populations; 4) with CXCR5 and CD25 for the identification of helper follicular cells and T regs; 5) with CXCR4 and CCR5 for HIV receptors; and 6) with CD8, CD45RO, CD38 and DR for the levels of activated and terminal cells. Red cells were lysed with Facs Lysis buffer (Becton Dickinson, Cowley, Oxford, UK) and analyzed in a Facscalibur flow cytometer.

### 4. Human IFN gamma and IL-2 ELISPOTS

Human IFN gamma and IL-2 ELISPOTS were detected as described previously. *See* Darwich L, et al., J. Acquir. Immune Defic. Syndr. 2008; 49(5):507-5712. Freshly isolated PBMCs were stimulated with either phytohaemmaglutinin (PHA, 5 µg/ml, Sigma-Aldrich, St. Louis, MO, USA), HIV-1 p24 and gp160 antigens (2.5 µg/ml and 5 µg/ml, respectively, Protein Science, Meriden, CT, USA), cytomegalovirus (10 µg/ml, Virion Serion, Walkersville, MD, USA), recombinant VHA proteins (2 µg/ml, Biodesign, Cincinnati, OH, USA), sAgVHB (5 µg/ml, Prospec, Rehovot, IL) or TT (10 µg/ml, Sigma-Aldrich, St. Louis, MO, USA ) on PVDF-bottomed well plates coated with anti-IFN-g and IL-2 (Diaclone, Besançon, France) for 48 hours. The INF-γ and IL-2 production was detected by a biotinylated anti-IFN-γ or IL-2 antibody followed by alkaline phosphate conjugate streptavidin (Amersham Biosciences, Uppsala, SE) visualized with a solution of 4-nitro-bleu tetrazolium chloride and 5-bromo-4 chloro-3-indolyl phosphate as recommended by the manufacturer (Sigma-Aldrich, St. Louis, MO, USA). A positive response was defined by an assay meeting the following criteria: 1) strong response in the PHA-stimulated positive control wells and 2) the detection of at least 50 spot forming units (SFU)/10⁶ PBMC after subtraction of non-stimulated cells. *See* Darwich *et al.*, 2008, *supra.*

### 5. Detection of HIV-specific CD8 T-cell responses

An epitope-specific ELISPOT assay was used to measure antigen-induced interferon-gamma (IFN-γ) release from CD8 T-cells. All analyses were performed on cryopreserved PBMC. A mean of 16 (range: 3-27) different HLA class I-restricted synthetic peptides from Gag, Pol, Env and Nef proteins were tested in each individual. Moreover, different pools of overlapping HIV-1 Gag 15-mer peptides derived from the sequence of strain HXB2 were also tested in parallel. Ninety-six well microtiter plates (Millipore, Billerica, MA, USA) were coated overnight with a mAb specific to human IFN-γ (mAb 1-D1K, Mabtech, Nacka Strand, SE). PBMC resuspended in RPMI plus 10% FCS were plated in the presence of different peptides at 4 µM and incubated overnight at 37°C, 5% CO2. Plates were developed using biotinylated anti-human IFN-y, streptavidin-bound phospatase alkaline conjugate and chromogenic substrate (BioRad Labs, Hercules, CA, USA). Spots forming cells (SFC) were counted using an AID ELISPOT reader (Autoimmun Diagnostika, Straßberg, DE). After subtracting background counts obtained with PBMC in medium alone, results were normalized to SFC/106 PBMC. A positive response was considered when counts were greater than 40 SFC/106.

### 6. Lymphoproliferative assay

PBMC were washed twice and resuspended at 2 x 10⁶/mL in serum-free medium X-VIVO 10 (BioWhittaker, Walkersville, MO, USA). Cultures were plated in triplicate at 2 x 10⁵/ well in 7 day assays, in 96 round-bottomed microplates (Coming Costar Europe, Schiphol-Rijk, NL). Cells were cultured in the absence or presence of 10µg/ml Pokeweed mitogen (PWM) (Sigma-Aldrich, St. Louis, MO, USA) as a polyclonal stimulus or 5 µg/mL of recombinant HIV-1 proteins gp160, and p24 (Protein Sciences, Meriden, CT, USA) with either phytohaemmaglutinin (PHA, 5 µg/ml, Sigma-Aldrich, St. Louis, MO, USA), HIV-1 p24 and gp160 antigens (2.5 µg/ml and 5 µg/ml, respectively, Protein Science, Meriden, CT, USA), cytomegalovirus (10 µg/ml, Virion Serion, MD, USA), live strain ref-AD-169 grown in human embryonic fibroblasts (CMV, 1:800 V/V, BioWhittaker, Walkersville, MO, USA), recombinant VHA proteins (2 µg/ml, Biodesign, Cincinnati, OH, USA), sAgVHB (5 µg/ml, Prospec, Rehovot, IL) or TT (10 µg/ml, Sigma-Aldrich, St. Louis, MO, USA). The incorporation of tritium-labelled thymidine during the last 18h of 7-day culture was evaluated using a betaplate (LKB, Wallac, FI). Results were expressed as mean counts per minute (cpm). The stimulation index (SI) was calculated for each sample as: cpm for cells minus the cpm of the spontaneous proliferation.

### 7. Proviral DNA

Genomic DNA from peripheral blood mononuclear cell (PBMC) pellets was extracted using the Puregene DNA isolation kit (QIAgen, Valencia, CA, USA). HIV *gag* sequences were amplified using a nested PCR using the following gag-specific primers: 5'-CTAGCAGTGGCGCCCGAACA-3' and 5'-ACAGTCTTTCATTTGGTGTCCTTC-3' for first-round outer PCR, and 5'-TCTCTCGACGCAGGACTC-3' and 5'-TTTCCACATTTCCAACAGCC-3' for second- round inner PCR. The PCR product was purified by PEG precipitation and either directly sequenced (referred to in the text as 'population sequencing'), or cloned using a TOPO TA cloning kit (Invitrogen, Carlsbad, CA, USA). Viral RNA was recovered from plasma samples using the Nucleospin RNA extraction kit (Mackery-Nagel, Düren, DE). During this step, RNA-free DNase (QIAgen, Valencia, CA, USA) was added to ensure removal of proviral DNA. A cDNA library was then synthesized from the RNA using the Reverse-iT 1st Strand Synthesis kit (ABgene, Waltham, MA, USA), using random oligonucleotides to prime the reaction. The *gag* fragments were then amplified and sequenced from the cDNA indicated above. All sequencing was done with BigDye Terminator v3.0 Ready Reaction mix (Applied Biosystems, Foster City, CA, USA), using the two inner PCR primers listed above and four additional primers (5'-CTGCACTATAGGATAATTTTGAC-3', 5'-GACACCAAGGAAGCCTTAG-3', 5'-CTCCCACTGGAACAGGTG-3' and 5'-GGAACAAATAGCATGGATGAC-3'). Sequences were analyzed on the ABI 3700 DNA analyzer. All residue numbers were taken against the HXB reference sequence.

### 8. Neutralization assay

Pseudo viruses were generated by co-transfecting 293T-cells with envelope (NL4-3 or AC10) expression plasmids and Δenv HIV-1 backbone vector (pSG3AEnv); 24h after transfection, culture supernatants were filtered (0.45µm) and stored at -80°C. TCID50 of pseudo viruses was determined in TZM-bl cells. Serial five-fold dilutions of pseudo virus were made in quadruplicate wells in a 96-well plate; 10.000 TZM-bl cells (containing 37,5µg DEAE-dextran/ml) were added in each well. After 48 hours, cultures were analyzed by illuminometer (Thermo Fisher Scientific, Waltham, MA, USA) using the Britelite Reagent (Britelite Luminescence Reporter Gene Assay System, PerkinElmer, Waltham, MA, USA) according to the manufacturer's instructions.

Neutralizing antibodies were measured as reductions in Luc Reporter gene expression after a single round of infection in TZM-bl cells. *See* Montefiori D, et al, J. Infect Dis. 2004; 190(11):1962-1969. The neutralizing antibodies against HIV, SIV and SHIV were evaluated in luciferase reporter gene assays. *See* Coligan J, Curr. Protoc. Immunol. 2001; Appendix 1: Appendix 1D.

200TCID₅₀ of pseudovirus were pre-incubated in a 96-well plate with various dilutions of heat-inactivated sera (starting dilution 1/50, fivefold stepwise) in duplicate for 1 hour at 37°C, 5% CO₂. TZM-bl cells were added at 10,000 cells/well containing 37.5µg DEAE-dextran/ml. Two days after the infection, cultures were analyzed by luminometry (Thermo Fisher Scientific, Waltham, MA, USA) using the Britelite Reagent (Britelite Luminescence Reporter Gene Assay System, PerkinElmer, Waltham, MA, USA) following the manufacturer's instructions. Then, neutralizing antibody titers (IC50), defined as the serum dilution required to reduce virus control RLU by 50%, were calculated (GraphPad Prism, GraphPad Software, La Jolla, CA, USA).

### 9. Statistical analysis

Data was analyzed with the PRISM statistical package. The Mann-Whitney test was used to compare groups. Spearman's correlation test was used to check the relationship between two sets of data. All tests of significance were two-tailed. Contingency tables were used to compare groups with categorical variables and were validated by means of Fisher's exact test.

Sixty five human subjects were tested for the presence of antibodies against hepatits A, B and tetanus toxoid. Of this amount, sixty three patients were vaccinated. Twenty-eight patients remained seronegative after vaccination. From this group, 21 patients were randomized in three groups: 1) group A (8 subjects), 2) group B (6 subjects) and 3) group C (7 subjects). Eight patients from group A, 4 from group B and 7 from group C completed the trial. *See* Figs. 1 and 2.

### Example 1: The administration of recombinant growth hormone results in an increase of thymic volume, levels of recent thymic emigrants and absolute CD4 and CD8 T-cell counts.

In order to assess whether the recombinant growth hormone had an effect on the volume or thymic tissue, a computerized axial tomography was performed at months 0, 6 and 12 after the initiation of the trial. Changes in volume, density and thymic index (TI) were reported.

From the 20 HIV+ patients assessed, 7 out of the 13 that received recombinant growth hormone (4 from group A and 3 from group B) showed a two-fold increase in the volume of the thymus at the end of the 6 months of treatment, while no relevant changes were observed in the untreated, control patients. Six months later, although the volume of the thymus had decreased, it was still significantly larger than before the initiation of the trial. The capacity of growth hormone to restore the thymus was not affected by any of the following parameters: age of the patients, CD4 nadir, CD4 at baseline, levels of IL-7, time between the CD4 nadir and the initiation of the trial or the level of IL-7 or IGF-1. Similarly, there was no significant correlation between any of the above parameters between responders and non responders in the patients that had received hormonal treatment (Groups A and B). *See* Fig. 3A.

In order to confirm that the thymic cells were exported to the periphery, we investigated whether there was an increase in the numbers of recent thymic emigrants in circulation by measuring changes in the levels of T-cell receptor excision circles (TRECs) and the absolute numbers of CD4, CD45RA and CD31 T-cells. Due to sample restrictions, the number of TRECs was calculated on whole PBMCs. However, there was a strong correlation between the level of TRECs and the number of the CD4, CD45RA and CD31 T-cells. *See* Fig. 3D. A statistical significant increase in recent thymic emigrants was shown with both markers in the patients that received recombinant growth hormone and those that did not. The increase was more striking in those subjects that experienced an increase in thymus volume, in response to hormonal treatment (TRECs 964 ±493 vs 249 ±108) (Fig. 3B). Similarly, there was also a statistically significant increase in the absolute numbers of CD4, CD45RA and CD31 T-cells in patients that had received rGH (median at 6 months 136 (56-193) vs 45 (7-86)) and those that had experienced an increase in thymic size (median at 6 months 154 (97-315) vs 25 (5-107) cells mm⁻³). *See* Fig. 3C.

Furthermore, the administration of recombinant growth hormone also induced an increase in the absolute numbers of CD4 T-cells and CD8 T-cells. This number was higher in the subjects with increased thymic tissue. *See* Figs. 4A and 4B. The levels of CD4 and CD8 T-cells from the vaccinated patients that were untreated (Group C) remained unchanged. *See* Figs. 4A and 4B. However, a phenotypic analysis was performed at months 0 (base line), 2, 4, 5, 6, 9 and 12 with the combinations of antibodies indicated above. The proportion of the different subpopulations was unaffected during the trial, except for an increase in the CD4RA recent thymic emigrants. *See* Figs. 4C and 4D. In summary, no statistically significant difference could be detected in the phenotype, proportion of NK, B and T-cells, in the percentage of naïve and memory cells, and in the levels of T regs on the helper follicular cells (CXCR5, CD57). The treatment did not affect the percentage of cells expressing the HIV co-receptors CXCR4 or CCR5. Similarly, the CD8 T-cell subsets remained also stable as measured by the expression of CD8, CD28, CD45RA, CD45RO and CD57, among other markers. No signs of lymphocyte activation were observed as measured by the expression of CD38, MHC Class II, CD69 or CD62L in any of the two populations. *See* Figs. 4C-4F.

### Example 2: The administration of recombinant growth hormone followed by vaccination induces the recovery of the CD4 specific responses to Hepatitis A/B, TT and HIV p24 in subjects that were undetectable at baseline.

Before the initiation of the trial, 18 HIV positive and 7 HIV negative subjects failed to elicit *in vitro* CD4 specific response to tetanus toxoid, hepatitis A and or hepatitis B as measured by the capacity of the CD4 T-cells to produce IFN gamma or IL-2 in the presence of these antigens (ELISPOT). From the 21 out of the 24 assays performed in group A (3 test per patient), 16 out of the 21 performed in group B and 16 out the 21 performed in group C were negative at base line. After the trials, 15 out of 21 in group A, 8 out of 15 in group B and 15 out of 20 in group C were now positive for these assays. There was a statistical significant differences between group A vs C p=0.001 and the controls and Group C, p=0.001, indicating that the patients from group A behaved very similarly to HIV negative patients in response to these antigens. Finally, 16 out of 21 tests from the HIV negative controls were undetectable for these antigens before vaccination; 14 out of the 16 became positive after vaccination.

The capacity of CD4 cell to release IL-2 when in contact with these antigens (Hep A, Hep B and TT) was evaluated. Only positive results were obtained when the cells were stimulated with tetanus toxoid, but the percentage of IL-2 producing cells was much lower than the number of cells that produced IFN gamma, as the majority of patients did not respond to the assay *in vitro.* However, 5 patients became positive for the IL-2 ELISPOTS to TT, 4 from group A and one from group B.

Next, whether the reconstitution of the thymus had a positive effect in the responses to HIV, measured by IFN gamma and IL-2 ELISPOTS to HIV p24 and HIV p160 was analyzed. Out of the original 21 tests performed, 16 were negative before the beginning of the trial. After the end of the trial, 5 out of 7 subjects in group A, 1 of 2 subjects in group B and 2 out of 7 subjects in group C showed positive responses. None of the HIV negative patients responded to p24 or gp160. *See* Fig. 5

To ensure that the lack of responses to CD4 were not due to a general defect of the patients to respond to foreign antigens, the responses to CMV was measured, as the majority of the HIV infected patients were positive for this antigen. Seventeen out of 21 patients responded to CMV before the initiation, as well as at the end, of the trial.

### Example 3: The restoration of the CD4 cellular responses elicits the production of antibodies to antigens that were previously seronegative.

After demonstrating that the treatment with recombinant growth hormone followed by vaccination induces CD4 specific responses, and, as CD4 T-cells are necessary for the induction of humoral responses, the seroconversion of the subjects that were previously seronegative to the antigens tested was considered. Five out of 8 patients in group A seroconverted to at least one antigen (one individual seroconverted to 2 antigens), 1 out of 5 of the subjects in group B and 2 out of seven of group C also elicited positive responses. Specifically, two patients from group A seroconverted for hepatitis A, and one to hepatitis B and two subjects showed at least a four-fold increase in the levels of antibodies to TT detected before the initiation of the trial. From group B, one patient seroconverted for hepatitis B and, from group C, two patients seroconverted for hepatitis A (see figure 6). All the patients that produced antibodies had also recovered the CD4 specific responses *in vivo*, confirming the need of CD4 helper responses for the production of antibodies.

Afterwards, whether recombinant growth hormone and the expansion of CD4 cell could affect the production of the neutralizing antibodies to HIV was also analyzed. Twenty patients were tested before and after the trial. Six out of the 20 showed titers of antibodies above 1/500. Nevertheless, none of the 14 resting patients had an increase in the level of neutralizing antibodies and the small changes detected were not statistically significant (p=0.84). (Group A and B, 294 ± 111; 302 ± 141 month 0 and 12 respectively, Group C, 498 ± 104; 1110 ± 452 month 0 and 12, respectively). The level of neutralizing antibodies from the HIV negative subjects were used as controls. None had detectable levels of antibodies.

### Example 4: The administration of GH did not increase the proviral HIV DNA and did not increase the plasma viral load.

Since HIV could infect the cells of the thymus, it would be possible that the augmentation of the thymus might result in an increase in the number of infected cells. Although all the patients were on HAART during the trial, the levels of proviral DNA could have increased, and subsequently increase the plasmatic viral load. However, there was no stastiscally significant difference in the proviral load at month 2, suggesting that GH did not increase the HIV plasmatic viral load (see figure 7).

## Claims

1. An agent capable of increasing the activity of growth hormone for use in a method of increasing the immune response against an antigen in a subject.

2. An agent for use according to claim 1 wherein the agent capable of increasing the activity of growth hormone is not a growth hormone secretagogue.

3. An agent for use according to claim 2 wherein the agent capable of increasing the activity of growth hormone is selected from the group consisting of:
(i) Growth hormone or a functionally equivalent variant thereof,
(ii) A polynucleotide encoding growth hormone or a functionally equivalent variant thereof and
(iii) A cell secreting growth hormone or a functionally equivalent variant thereof.

4. An agent for use according to any of claims 1 to to 3 wherein said method comprises the administration of a vaccine composition comprising said antigen.

5. An agent for use according to any of claims 1 to 5 wherein the antigen is a viral antigen.

6. An agent according to claim 5 wherein the viral antigen is selected from the group of a hepatitis A virus antigen, a hepatitis C antigen and a HIV-1 antigen.

7. An agent for use according to any of claims 1 to 4 wherein the subject which receives the therapy is immunosupresssed.

8. An agent for use according to any of claims 1 to 5 wherein said agent is chronically administered.

9. An agent for use according to any of claims 1 to 6 wherein the agent is administered at a dosage that results in IGF-1 serum levels of equal or lower than 500 U/ml.

10. An agent for use according to any of claims 1 to 7 wherein the antigen is selected from the group of a cancer antigen, a bacterial antigen, a viral antigen and an allergen.

11. A composition or kit comprising
(i) An agent capable of increasing the activity of growth hormone and
(ii) An antigen in amounts sufficient to induce an immune response against said antigen.

12. A composition or kit according to claim 9 wherein the agent is not a growth hormone secretagogue.

13. A composition or kit according to claim 10 wherein the agent is selected from the group consisting of:
(i) Growth hormone or a functionally equivalent variant thereof
(ii) A polynucleotide encoding growth hormone or a functionally equivalent variant thereof
(iii) A cell secreting growth hormone or a functionally equivalent variant thereof

14. A composition or kit according to any of claims 9 to 11 wherein the antigen is selected from the group of a cancer antigen, a bacterial antigen, a viral antigen and an allergen.

15. A composition or kit according to any of claims 9 to 12 for use in medicine.

16. A composition or kit according to any of claims 9 to 12 for use as a vaccine.

17. A composition or kit according to claims 13 or 14 wherein components (i) and (ii) are simultaneously, sequentially or separately administered.


SEQUENCE LISTING

<110>  Laboratorios del Dr. Esteve S.A.
       Fundació Privada Institut de Recerca SIDA-Caixa

<120>  USE OF GROWTH HORMONE TO ENHANCE IMMUNE RESPONSE IN
       IMMUNSUPRESSED PATIENTS

<130>  P6306EP00

<160>  8

<170>  PatentIn version 3.5

<210>  1
<211>  20
<212>  DNA
<213>  Artificial

<220>
<223>  gag primer

<400>  1
ctagcagtgg cgcccgaaca                                                   20


<210>  2
<211>  24
<212>  DNA
<213>  Artificial

<220>
<223>  gag primer

<400>  2
acagtctttc atttggtgtc cttc                                              24


<210>  3
<211>  18
<212>  DNA
<213>  Artificial

<220>
<223>  gag primer

<400>  3
tctctcgacg caggactc                                                     18


<210>  4
<211>  20
<212>  DNA
<213>  Artificial

<220>
<223>  gag primer

<400>  4
tttccacatt tccaacagcc                                                   20


<210>  5
<211>  23
<212>  DNA
<213>  Artificial

<220>
<223>  gag primer

<400>  5
ctgcactata ggataatttt gac                                               23


<210>  6
<211>  19
<212>  DNA
<213>  Artificial

<220>
<223>  gag primer

<400>  6
gacaccaagg aagccttag                                                    19


<210>  7
<211>  18
<212>  DNA
<213>  Artificial

<220>
<223>  gag primer

<400>  7
ctcccactgg aacaggtg                                                     18


<210>  8
<211>  21
<212>  DNA
<213>  Artificial

<220>
<223>  gag primer

<400>  8
ggaacaaata gcatggatga c                                                 21